# EUROPÄISCHE PATENTSCHRIFT

(11) **EP 1 127 274 B1**
(45) Veröffentlichungstag und Bekanntmachung des Hinweises auf die Patenterteilung: **07.10.2009**
(21) Anmeldenummer: 00943855.7
(22) Anmeldetag: 21.06.2000
(51) Int. Cl.: G01N 33/18

(54) **DETEKTOREN UND IHRE ANWENDUNG**
DETECTORS AND THEIR USE
DETECTEURS ET LEUR UTILISATION

(30) Priorität: 24.06.1999 DE 19928460; 05.07.1999 DE 19930764; 02.10.1999 DE 19947635; 05.01.2000 DE 10000269; 29.01.2000 DE 10003841; 09.04.2000 DE 10017304; 10.05.2000 DE 10022549
(43) Veröffentlichungstag der Anmeldung: 29.08.2001
(73) Patentinhaber: Oeste, Franz-Dietrich, 35274 Kirchhain (DE)
(72) Erfinder: Haas, Rainer, 35037 Marburg (DE); Oeste, Franz-Dietrich, 35274 Kirchhain-Schönbach (DE)
(86) Internationale Anmeldenummer: PCT/EP2000/005745
(87) Internationale Veröffentlichungsnummer: WO 2001/001126

(56) Entgegenhaltungen:
- DE-A- 2 403 971
- DE-A- 2 640 695
- DE-A- 19 643 817
- DE-A- 19 726 813
- DE-A- 19 834 916
- US-A- 4 125 376
- US-A- 5 355 736
- US-A- 5 482 773
- US-A- 5 851 613

## Beschreibung

Beschrieben werden Detektoren und Verfahren zur Detektion von Stoffen in Flüssigkeiten zur Analyse in Meßstellen zur Wasser-, Boden- und Sediment-Untersuchung auf natürliche und künstliche Inhaltsstoffe inklusiv radioaktiver Kontaminanten, einschließlich ihrer Probenahme, zur Überwachung und Probenahme in der Lebensmittel- und Futtermittelerzeugung sowie von Industrieprodukten, industriellen Prozessen und im Kernenergiesektor sowie zur Anwendung auf einer Reihe weiterer Gebiete. Weiterhin wird die Anwendung des Analysen-Verfahrens auf die Lebensmittel- und Futtermittel-Überwachung sowie die Überwachung von Industrieprodukten beschrieben. Das Verfahren erstreckt sich auch auf die Überwachung von nicht-wäßrigen Flüssigkeiten und von feuchtem Erdreich und Sedimenten.

Zum Entfernen von Stoffen aus Flüssigkeiten sind seit langem Filtereinrichtungen üblich, die nach unterschiedlichen Prinzipien arbeiten, z.B. mit mechanischen oder sorptiven Mitteln. Um die Betriebskosten niedrig zu halten, müssen sorptive Filter eine Reihe von Anforderungen erfüllen, wie Minimierung des spezifischen Filtervolumens und Maximierung des Flüssigkeitsdurchsatzes pro Zeiteinheit sowie der spezifischen Beladungskapazität. Durch die Maximierung des spezifischen Flüssigkeitsdurchsatzes wird ein möglichst geringer Abstand der Sorbensteilchen voneinander erreicht; dadurch wird die zu reinigende Flüssigkeit gezwungen, sich so dicht wie möglich an der adhärierenden Phase der Sorbens-Oberfläche des Filters zu bewegen. Entsprechend der kurzen notwendigen Aufenthaltsdauer im Filter kann der Flüssigkeitsdurchsatz gesteigert werden. Durch die Minimierung des spezifischen Filtervolumens wird möglichst viel Sorbens hineingepackt. Übliche spezifische Sorbens-Packungsdichten bewegen sich im Bereich von 50 bis 500 g/ℓ und darüber. Die spezifischen Beladungskapazitäten des Filters, in der Regel 1 bis 20% der Sorbens-Masse, betragen bei konventionellen Filtern z.B. 0,5 bis 100 g/ℓ und darüber.

Detektoren zur Untersuchung von Stoffen in Flüssigkeiten unterscheiden sich in mehrerer Hinsicht von Filtereinrichtungen oder von Vorrichtungen zur Entfernung von Stoffen. Zur wirksamen Entfernung aus einer gegebenen Flüssigkeitsmenge müssen die zu behandelnden Flüssigkeiten möglichst dicht an den Filtereinrichtungen vorbeigeführt werden. So wird angestrebt, diese Filterelemente im Flüssigkeitsstrom möglichst dicht zu packen, um deren gegenseitigen Abstand und damit das Zwickelvolumen, durch das die Flüssigkeit bei der Reinigung hindurchtritt, möglichst gering zu bemessen; dies hat einen erheblichen Strömungswiderstand zwischen Flüssigkeit und Filter zur Folge. Im Gegensatz dazu dürfen Detektoren der strömenden Flüssigkeit nur einen minimalen Widerstand entgegensetzen; diese werden in einem offenen System eingesetzt und es muß vermieden werden, daß die Flüssigkeit um die Detektoren herumströmt.

Ein weiterer Unterschied zwischen Filterelementen und Detektoren besteht darin, daß erstere die Aufgabe haben, den gewünschten Stoff möglichst vollständig aus der Flüssigkeit zu entfernen, während letztere die Zusammensetzung der zu untersuchenden Flüssigkeit praktisch nicht verändern oder gezielt Stoffe in die Flüssigkeit abgeben sollen. Diese Eigenschaften können durch entsprechende Auswahl von Wirkstoffart und -menge im Detektor eingestellt werden.

Nachfolgend wird der Stand der Technik zur Untersuchung von Stoffzuständen skizziert. Insbesondere natürliche, aber auch künstliche bzw. technische Kompartimente sind grundsätzlich von heterogenem Zustand, d.h. sie haben an unterschiedlichen Orten eine unterschiedliche Stoffzusammensetzung. Das gilt sowohl fur feste als auch für fluide Stoffe, wie Flüssigkeiten, Erdreich oder Sedimente. Gleichermaßen bezieht sich die Ungleichförmigkeit der Stoffzustände auch auf den zeitlichen Maßstab, da sich vor allem durch Bewegung der Fluide deren Zusammensetzung an einem gegebenen Ort ständig ändert. Insbesondere natürliche Stoffe unterliegen einem Wandel der Stoffzusammensetzung. Je ausgedehnter das zu untersuchende Kompartiment ist, desto weiter differiert in der Regel seine Stoffzusammensetzung.

Um den Stoffzustand von Kompartimenten zu ermitteln, wurden Probenahme- und Analysenverfahren entwickelt, üblicherweise auf wenige stichprobenhafte Analysen beschränkt, die zur Ermittlung der durchschnittlichen Zusammensetzung des untersuchten Kompartiments ausreichten. Dies mochte für technische Produkte und Stoffströme mit geringen örtlichen oder zeitlichen Gradienten der Stoffzusammensetzung ausreichen, wie sie z. B. für den Inhalt eines Öltanks gelten. Die Notwendigkeit der Abbildung der extrem heterogenen Stoffverteilung und ihre Änderung im Zeitverlauf, wie sie z. B. im Grundwasseraquifer um einen kontaminierten Altstandort auftritt, erzwingt jedoch, solche Probenahme- und Analysenmethoden nicht zu benutzen. Derartig heterogene Systeme lassen sich mit den Analysensystemen nach dem Stand der Technik nur mangelhaft abbilden.

Ein anderes Kompartiment, dessen Zusammensetzung wegen der Gefährdung von Mensch und Umwelt möglichst genau abzubilden ist, ist in die Gewässer eingeleitetes Abwasser, dessen Zusammensetzung sich extrem schnell ändern kann. Infolgedessen kommt es immer wieder zu Schäden an den natürlichen Gewässern durch Einleitung toxischer Stoffe. Die herkömmliche Praxis der Entnahme von Einzelproben oder Mischproben ist nicht in der Lage, die zeitliche Entwicklung von Konzentration und Abwasservolumen lückenlos abzubilden.

Insbesondere in natürlichen Gewässern, darunter vor allem den Grundwasseraquiferen, sind neben dem stofflichen Zustand des Aquifers auch seine momentanen, vergangenen und daraus entwickelt seine zukünftig zu erwartenden hydraulischen Bewegungen abzubilden, um z. B. die zu erwartende Ausbreitung von Kontaminanten abschätzen zu können. Hier herrschen nach dem Stand der Technik insbesondere zur Gewinnung entsprechender Parameter von Grundwasseraquiferen noch recht einfache Verfahren anhand von Aquiferspiegelmessungen nach dem Abpumpen zur Ermittlung von Strömungsrichtung und -geschwindigkeit vor, die an mindestens drei Meßpegeln vorgenommen werden müssen. Mittels kamera-optischer Beobachtung von Schwebeteilchen lassen sich mittlerweile an einzelnen Lokalitäten Strömungsgeschwindigkeit und -richtung im Aquifer bestimmen. Die Ergebnisse auch dieser Untersuchungen sind kaum dazu geeignet, das komplexe Strömungsschema derartiger Aquifere innerhalb eines Beobachtungszeitraumes gleichzeitig an vielen unterschiedlichen Stellen abzubilden.

Die Gewinnung der zu analysierenden Proben oder Rückstellproben von Fluiden, z.B. Abwasser, Grundwasser, flüssigen Lebensmitteln oder Trinkwasser sowie der Proben-Transport in ein Labor zur Untersuchung oder als Rückstellmuster in ein Lager oder die Proben-Zwischeniagerung im Kühlraum sind sehr zeitaufwendig, personalintensiv, benötigen viel Lagerkapazität und sind daher sehr kostenintensiv. Aus diesen Gründen ist es bei den herkömmlichen Untersuchungsmethoden geboten, die Anzahl der Proben einzugrenzen. Eine lückenlose bzw. wünschenswert große Untersuchungs-oder Rückstell-Probenmenge ist deshalb nicht praktikabel.

Es gibt bereits eine Reihe von Meßverfahren, die insbesondere auch Stoff- und Stoffkonzentrations-Detektionsmittel auf Fasermaterial-Basis verwenden. So gibt es für eine Reihe von Stoffnachweisen Teststäbchen, die als Meßergebnis ein optisch erkennbares farbiges Reaktionsprodukt enthalten, das sich durch Wasserunlöslichkeit auszeichnet. Die Teststäbchen enthalten den Detektionswirkstoff ausblutungssicher in chemisch an die Oberfläche des Detektorträgers gebundener Form.

Daneben werden Papierstreifen angeboten, die als Meßergebnis ebenfalls optisch erkennbare farbige Reaktionsprodukte enthalten, die aber wegen deren Wasser-löslichkeit weniger zuverlässige Ergebnisse liefern. Damit lassen sich Oxidationsmittel, Schwefelwasserstoff und Wasserstoffionen-Konzentration nachweisen.

Die bekannten auf Papierbasis beruhenden Detektoren eignen sich aus einem oder mehreren der Gründe Form (Meßstäbchen mit Meßfeld), ihrer Wasser-Löslichkeit der reaktiven Substanzen (Papierstreifen) und der Unbeständigkeit der farbgebenden Substanzen (Papierstreifen) nicht für langfristige Messungen in wäßrigen Medien. Deshalb sind solche Detektoren auch nicht für die lückenlose Bestimmung von Stoffparametern auf einem ununterbrochenen Linienabschnitt, auf einem ununterbrochenen Flächenanteil oder in einem lückenlosen Volumenraum oder über eine längere Zeitdauer geeignet. Die Speicherkapazität der genannten Teststäbchen- und Papier-Streifen-Detektionseinrichtungen für den zu detektierenden Stoff ist minimal. Selbst wenn das Detektions-Produkt nicht ausblutet, sind sie fur die Langzeitmessung völlig unbrauchbar, weil sie nur über eine extrem geringe Beladungskapazität für den zu detektierenden Stoff verfügen. Diese Detektoren liefern also lediglich Ergebnisse über die momentane Konzentration von Stoffen z.B. in einer bereits entnommenen Wasserprobe, sie sind aber nicht für die Stoffmassenbilanzierung geeignet.

Herkömmliche Meßverfahren zur Ermittlung von Strömungsbedingungen beruhen auf der direkten Detektion der Strömung mit mechanischen Detektoren, bei denen die Strömung z.B. mit beweglichen Drehflügeln oder unter Nutzung des Coriolis-Prinzips gemessen wird. Diese Meßprizipien eignen sich jedoch nur für schnelle Strömungsvorgänge, die bei vielen Anwendungen in natürlichen Gewässern nicht auftreten.

Eine andere Möglichkeit beruht auf der Zugabe von Markierungsstoffen als Detektor, z.B. von optisch detektierbaren Markern wie Eosin, Salzlösungen oder radioaktiven Substanzen. Oft müssen erhebliche Mengen dieser Stoffe in die zu vermessenden Aquifere hineingegeben werden, um zu verwertbaren Ergebnissen zu kommen. Zudem besitzen viele der verwendeten Markierungsstoff-Detektionsmittel toxisches oder zumindest ein umweltschädliches Potential.

Vielfach wird die Strömungsrichtung in Grundwasseraquiferen auch durch Vermessung der Spiegelhöhen in Grundwasserpegeln ermittelt. Dazu sind mindestens drei Pegel notwendig. Diese Methode ist sehr teuer, wenn eigens zu diesem Zweck Pegel installiert werden müssen, und sie ist unzureichend, wenn beispielsweise die detaillierte Strömungsstruktur als vertikales Profil ermittelt werden soll oder wenn es um die kleinräumige Ermittlung von Strömungsstrukturen in oberflächennahem Schottern und Kies von Fließgewässern geht. Insbesondere in der Nähe von Flüssen ist diese Methode untauglich, da sich je nach Wasserstand des benachbarten Flusses die Strömung im Grundwasseraquifer ändern und sogar umkehren kann.

Die Fließgeschwindigkeit von Grundwasserströmen läßt sich bislang nur indirekt aus dem Aquifergefälle und der Durchlässigkeit des Aquifergesteins berechnen. Anhaltspunkte zur Gesteinsdurchlässigkeit ergeben Grundwasser-Spiegelabsenkungen beim Abpumpen gegebener Wassermengen und der Verlauf des Grundwasserspiegelanstiegs im Pegel nach beendetem Abpumpen. Die daraus gewonnenen Ergebnisse sind sehr vage und erfassen die Verhältnisse über den gesamten Aquiferquerschnitt, sie geben jedoch keinerlei Auskunft über die Strömungsverhältnisse im Detail. Hier fehlen einfache und zuverlässige Methoden.

Neben der Ermittlung der Fließstrukturen in einem Gewässer ist es für viele Untersuchungen notwendig, das physikalisch-chemische und ggf. biologische Milieu im Gewässer festzustellen, z.B. Temperatur, pH-Wert, Leitfähigkeit, Redox-Potential, Sauerstoff, Eisen, Mangan, Nitrat, Sulfat, Methan, Kohlensäure/Kohlendioxid, Erdalkalien, Ammonium, Hydrogensulfid/Schwefelwasserstoff, Huminstoffe, Vorkommen von Enzymen, Mikrobenspezies und Mehrzellerspezies. Darüber hinaus ist es oftmals wichtig, bestimmte Kontaminanten oder Kontaminantengruppen zu ermitteln, so z.B. Halogenorganika, die ein-und mehrkernigen Aromaten, die Nitro-und Aminoaromaten und die Biozide. Diese Kontaminanten sind oft nicht homogen im gesamten Aquifer vorhanden, etwa wenn Konzentrationsunterschiede im Grundwasser bestehen.

Durch die herkömmlichen Probenahmetechniken, nämlich Abpumpen der Probe zur Detektion der Parameter, werden viele der genannten Parameter so massiv verfälscht, daß sie nicht oder nur eingeschränkt zur Auswertung herangezogen werden können. Auch ist es schwierig, die Konzentrationen bestimmter Kontaminanten in definierten Tiefen im Wasser einer Meßstelle zu bestimmen, da durch das Pumpen ein Sog entsteht und evtl. Konzentrationsunterschiede gestört werden.

In DE-A-196 43 817 wird ein Verfahren zur sensitiven und korrekten Ermittlung der Gewässerparameter mitgeteilt. Dabei erfolgt die in-situ-Messung der Bewegungsparameter Fließgeschwindigkeit und Richtung sowie auch der chemischen Parameter mit einer miniaturisierten Detektionseinrichtung auf kleinem Raum. Die Detektionsvorrichtung wird durch das Gewässer bewegt. Durch die Detektion an verschiedenen Stellen im freien Gewässer kann damit ein dreidimensionales Parametermuster des Gewässermilieus erstellt werden. Auch in Meßpegeln zur Grundwasser-Messung kann diese Methode eingesetzt werden. Dies ist aber sehr zeit-und arbeitsaufwendig, da an vielen Meßpunkten Untersuchungen vor Ort durchgeführt werden müssen. Es wird daher nach Detektions-Verfahren und Detektoren gesucht, mit denen mindestens die gleiche, vorzugsweise aber hohe Parameterdichte darstellbar ist, jedoch der im Vergleich zu reinen Labormessungen vielfach höhere Meßaufwand vor Ort ganz vermieden werden kann.

DE-A-196 43 817 beschreibt ein Verfahren zum Ermitteln von Strömungsparametern in einem Fluid, wobei angestrebt wird, eine Beeinträchtigung der Umwelt durch strahlungsemittierende Meßstoffe, durch nichtstrahlende Meßstoffe wie Elektrolyte und durch Lösungen zur Zementation zu vermeiden, indem man die Meßstoff-Emissionsquelle und den Detektionsort innerhalb des Meßgeräts anordnet, auch unter Verwendung eines quantitativ sorbierenden Sorptionsbettes. Das Gewässer muß mit künstlichem Meßstoff angereichert werden.

Die DE-A-26 40 695 befaßt sich mit Indikatoren auf der Basis von Wollfarbstoffen, wobei das Trägermaterial ein in Wasser kaum oder nicht löslicher Eiweißkörper ist. Die Indikatormaterialien können pH-, Redox- oder Metall-Indikatoren dienen.

US-A-4 125 376 offenbart ein Detektionsverfahren mit einem Proberöhrchen, in dem ein Testschwammwürfel liegt, der mit einem Indikatomaterial imprägniert ist, z.B. einem Schwermetall- oder Phenolindikator. Die Methode bezweckt einen Schnelltest binnen weniger Minuten.

Gemäß DE-A-198 34 916 sind bereits Wirkstoffe enthaltende Fasermaterialien etwa bei der Wasserbehandlung eingesetzt worden, um die Eigenschaften von Wässern zu verändern, Schadstoffe zu entfernen oder den Geschmack von Lebensmitteln zu verbessern. Wirkstoffhaltige Fasergebilde und ihre Herstellung sind auch Gegenstand der WO-A-00/16877, und zwar als Wasser-Behandlungsmittel. Maßgeblich sind dort Ausbeute und Produktreinheit.

Aus der DE-A-198 34 916 und WO-A-00/16877 sind bereits wirkstoffenthaltende Fasermaterialien bekannt, die zur Wasserbehandlung eingesetzt werden. Mit diesen Fasermaterialien wird eine Verbesserung der Eigenschaften von Flüssigkeiten erreicht, beispielsweise Schadstoffe entfernt oder der Geschmack von Lebensmitteln verbessert. Die Mittel sind also zur Entfernung von Stoffen aus Flüssigkeiten vorgesehen. Gemäß WO-A-00/16877 können die wirkstoffaufweisenden Trägermaterialien auch, an Grundankern oder Bojen verankert oder eingebracht in Körbe, in einen Wasserkörper ausgesetzt werden, um dessen Zusammensetzung zu ermitteln.

In der US-A-5,355,736 wird ein Detektor zur Gewässeranalyse beschrieben, der eine sphärische, wasserdurchlässige Einhausung aufweist, die ein partikuläres Adsorbermaterial, beispielsweise ein lonentauschermaterial, enthält.

Aus den Anforderungen nach einer möglichst hochauflösenden Abbildung der Verteilung eines zu detektierenden Stoffes und/oder der Abbildung einer Strömungsstruktur innerhalb eines offenen Untersuchungskompartiments ergeben sich eine Reihe von Anforderungen, die einzeln oder in Kombination durch die erfindungsgemäßen Detektoren erfüllt werden.

So soll ein zu detektierendes Aquifer durch die Probenahme möglichst wenig gestört werden, um die dort zu untersuchenden Verhältnisse korrekt abbilden zu können. Ferner soll der Detektor eine hinreichende Aufnahmekapazität aufweisen, um die zu detektierende Substanz über einen vorbestimmten Zeitraum anzusammeln. Auch soll der Detektor einen möglichst geringen hydraulischen Widerstand besitzen, um die Strömungsverhältnisse im gesamten Einflußbereich des Detektors abzubilden. Ferner soll die zu detektierende Substanz sich möglichst homogen auf dem Detektor verteilen, und die Detektoren sollen eine Reihe von Wirkstoffen enthalten können, die in üblichen Flüssigkeitsfiltern nicht vorkommen.

Es ist also höchst erstrebenswert, nach einem Detektionsverfahren zu suchen, das bei vermindertem Probenahmeaufwand, kleinerer Personal- und Lagerkapazität und erheblich geringerem Zeitaufwand eine zeitlich und örtlich lückenlose Probenahme sowie eine bessere Probenqualität ermöglicht, wobei in das gewonnene Detektionsergebnis die Parameter Stoffkonzentration des oder der zu messenden Stoffe, die Masse des durch den Einflußbereich des Detektors bewegten Fluides und die gewählte Zeitdauer des Detektionsvorganges Einfluß nehmen. Die Aufgabenstellung besteht weiter darin, verbesserte Möglichkeiten zur gesteigerten raum-zeitlichen Informationsdichte über die jeweils wünschenswerten Analysenparameter und dafür geeignete Probenahmetechniken zu finden, die es auch ermöglichen sollen, die Massenbilanzierung von einzelnen Stoffen und gegebenenfalls Stoffinventaren vorzunehmen.

Hauptmerkmale der Erfindung sind in den Ansprüchen 1 und 7 angebeben. Ausgestaltungen sind Gegenstand der Ansprüche 2 bis 6 und 8 bis 11.

Bei einem Detektor im offenen System zur Ermittlung der Art und/oder des Gehalts von vorbestimmten Substanzen und/oder zur Ermittlung von Richtungs- und/oder von Geschwindigkeits-Parametern an einem Untersuchungsort in Gewässern und fluiden dichten Medien, wobei das Medium während der Untersuchungsdauer, nämlich einem vorbestimmten Zeitraum von mehr als einer Stunde, aktiv oder passiv ein Volumenelement des im offenen System angeordneten Detektors um- oder durchströmt, wobei dieser aus einem wenigstens einen Wirkstoff aufweisenden Trägermaterial, beispielsweise Papier, Textil, Folie, Streifen, Band, Faden, Draht, Stab, Wolle, Partikel o.dgl. besteht, wobei der bzw. jeder Wirkstoff auf dem Trägermaterial angeordnet und/oder darin enthalten und im Untersuchungsmedium durch Einwirkung vorbestimmter Substanzen, die am Untersuchungsort vorkommen, auf vorbestimmte Weise veränderbar ist und wobei der bzw. jeder Wirkstoff in einer für die Untersuchungsdauer ausreichenden Menge vorhanden ist, sieht die Erfindung laut Anspruch 1 vor, daß die Packungsdichte des Trägermaterials im offenen System weniger als 100 g/ℓ beträgt, daß die Ausdehnung des Detektors im Untersuchungsmedium in einer Raumdimension gegenüber seinen Ausdehnungen in den beiden anderen Raumdimensionen überwiegt oder daß seine Ausdehnungen im Untersuchungsmedium in zwei Raumdimensionen gegenüber seiner Ausdehnung in der dritten Raumdimension überwiegen und daß der Detektor nur schwerlösliche oder unlösliche Wirkstoffe enthält und im Untersuchungsmedium in Form einer Anordnung oder Dispersion von partikulären und/oder faserförmigen Teilchen vorliegt.

Ein Verfahren im offenen System zur Detektion der Art und/oder des Gehalts von vorbestimmten Substanzen und/oder zur Ermittlung von Richtungs- und/oder von Geschwindigkeits-Parametern an einem Untersuchungsort in Gewässern und fluiden dichten Medien, wobei das Medium während der Untersuchungsdauer ein Volumenelement eines frei oder in offener Einhausung angeordneten Detektors nach einem der Ansprüche 1 bis 14 während mehr als einer Stunde aktiv oder passiv um- oder durchströmt und der Detektor aus einem wenigstens einen Wirkstoff aufweisenden Trägermaterial, beispielsweise Papier, Textil, Folie, Streifen, Band, Faden, Draht, Stab, Wolle, Partikel o.dgl. besteht, wobei der bzw. jeder Wirkstoff auf dem Trägermaterial angeordnet und/oder darin enthalten und im Untersuchungsmedium durch Einwirkung vorbestimmter Substanzen, die am Untersuchungsort vorkommen, auf vorbestimmte Weise veränderbar ist und wobei der bzw. jeder Wirkstoff in einer für die Untersuchungsdauer ausreichenden Menge vorhanden ist, besteht laut Anspruch 15 darin, daß der Detektor im Wege der passiven Probenahme in das Untersuchungsmedium eingebracht und darin während der Untersuchungsdauer belassen wird, worauf der Detektor entnommen wird und die Bestimmung der Menge des Wirkstoffs und/oder der durch Einwirkung der vorbestimmten Substanz und/oder der Strömung des Flüssigkeitsstroms auf den Wirkstoff entstandenen Reaktionsprodukts als Maß für die Art und gegebenenfalls die Menge der vorbestimmten Substanz und/oder der Strömung des Flüssigkeitsstroms erfolgt.

Das erfindungsgemäße Verfahren beruht auf der Bildung oder Entfernung unter geeigneten Bedingungen dauerhaft beständiger und detektierbarer Substanzen am Detektor als Ergebnis mindestens einer chemischen und/oder physikalischen Reaktion von flüssigen, insbesondere wäßrigen Stoffen mit Wirkstoffen. Die Detektion erfolgt innerhalb des unverändert belassenen Vorkommens der zu untersuchenden Flüssigkeit, mit Ausnahme der durch die Detektion und die Maßnahmen zu ihrer Durchführung verursachten Störungen, also in Form einer "passiven Probenahme".

Der erfindungsgemäße Detektor befindet sich im Gegensatz zu Sorptionsfiltern in einem offenen System. Daraus ergibt sich die Forderung, insbesondere bei geringfügigen Strömungen, einen genügend weiten Abstand der Träger- und/oder Wirkstoffteilchen bzw. der Detektoreinheiten voneinander zu wählen, um die zu detektierenden Stoffe nicht zu veranlassen, sich um den Einflußbereich des Detektors herum zu bewegen. Weil der Detektor üblicherweise die zu untersuchende Substanz auf seiner Oberfläche oder im Innern ansammelt, ist neben der Konzentration des zu detektierenden Stoffes in der Flüssigkeit auch die Menge der durch den Detektor hindurchströmenden Flüssigkeit ausschlaggebend. Da das vorhandene Strömungsmuster durch die Anwesenheit des Detektors nicht gestört werden soll, ist er mit einer möglichst geringen Fläche und/oder Volumen auszugestalten; das Volumen des Detektors hat der Flüssigkeitsströmung einen möglichst geringen Widerstand zu bieten. Diese Eigenschaften können durch eine geeignete Auswahl der Packungs- oder Schüttdichte des wirkstoffbeladenen Trägers oder des Wirkstoffes (der gleichzeitig Träger sein kann) erreicht werden. Üblicherweise bewegt sich die Packungsdichte zwischen 1 und 100 g/t bei Fasern oder Wollen (z. B. aus Papier oder Folien). Bevorzugt sind die Wollen für die schlauch-oder zylinderformigen Detektoren mit einem Durchmesser von 0,5 mm bis 100 mm. Partikelschüttungen werden bevorzugt eingesetzt für schlauchzylinderförmige Detektoren mit Durchmessern von 5 bis 15 mm. Die schlauch- oder zylinderförmigen Detektoren enthalten bevorzugt netzschlauchformige Einhausungen, und sie können anstelle der Schüttung oder Packung auch zur Einhausung von bandförmigem Material eingesetzt werden. Flächenfüllende Detektoren sind vorzugsweise nicht breiter als 10 mm, um stauende Umströmung zu minimieren.

Anders als bei Filtereinrichtungen entnehmen die Detektoren beim Detektionsvorgang nur einen geringen Anteil des in der gesamten Flüssigkeit vorhandenen Stoffes. Die Größe dieses Anteils ist je nach Anwendung von Fall zu Fall verschieden. Die Detektoren gestatten üblicherweise neben der Bestimmung der Art des zu untersuchenden Stoffes auch die Bestimmung von dessen gesammelter Menge (integrierende Untersuchung). Die Aufnahmekapazität des Detektors und somit sein Gehalt an Wirkstoff muß daher ausreichen, um die Aufnahme des zu detektierenden Stoffes über einen längeren Zeitraum zu gewährleisten.

Da sich der Detektor beim Einsatz in einem offenen Strömungssystem befindet, können bereits geringe hydraulische Widerstände erheblichen Einfluß auf das Strömungsverhalten der Flüssigkeit ausüben. Ein Detektor zur Vermessung von Strömungsdaten mit erheblichem Querschnitt bzw. erheblicher Raumfüllung muß daher möglichst permeabel fur die Flüssigkeit gehalten werden, beispielsweise durch eine niedrige Schütt-oder Packungsdichte des Trägers und/oder des Wirkstoffes.

Für eine möglichst genaue Analyse der Meßdaten ist es erwünscht, daß der Detektor über die gesamte Meßfläche den zu untersuchenden Stoff möglichst homogen abbildet. Insbesondere bei inhomogenen und/oder dichten Packungen kann ein Risiko bestehen, daß sich die zu detektierende Substanz in unterschiedlicher Menge in einzelnen Regionen des Detektors niederschlägt, was insbesondere dann Meßergebnisse verfälschen kann, wenn die Detektoren zur Analyse geteilt werden.

Die erfindungsgemäßen Detektoren können sowohl passiv als auch aktiv von der zu detektierenden Flüssigkeit des offenen Untersuchungskompartiments durchströmt werden. Die passiv durchströmten Typen werden bevorzugt, mit denen Strömungs- und/oder Kontaminationsverteilungen ermittelt werden können. Aktiv durchströmte Detektoren dienen vorzugsweise der Spurenstoff-Detektion oder auch dazu, Gleichgewichtskonzentrationen für bestimmte Stoffe vor Ort aufzunehmen.

Die jeweils angewendeten Detektoren sind in dem kompletten zu untersuchenden, punkt-, linien-, fläche- oder volumenförmigen Kompartiment als solche in ihrer Gesamtheit ständig oder zumindest während eines kurzen Zeitabschnittes vorhanden. Das erfindungsgemäße Verfahren ist zumindest for die Untersuchung punktförmiger Kompartimente auf Zeitabschnitte ausgelegt, die mindestens mehr als eine Stunde umfassen. Die das Untersuchungsverfahren ermöglichenden erfindungsgemäßen Detektoren erfüllen damit das Ziel, die An- und/oder Abwesenheit bestimmter Stoffe und/oder Stoffzustände und/oder ihre Bewegung während der Detektionsdauer am Detektionsort zeitlich und/oder örtlich lückenlos abzubilden sowie dauerhaft und jederzeit abrufbar aufzuzeichnen. Als Ergebnis dieser Reaktionen werden damit in ihrer stofflichen Zusammensetzung dauerhaft und in typischer Weise veränderte Detektoren erhalten, die geeignet sind, die während der Dauer der Messung und an den vermessenen Ortskoordinaten vorhandenen bestimmten Stoffe und/oder Stoffzustände und/oder ihre Bewegung auf Dauer zu dokumentieren. Die in den Detektoren nach dem beendeten Detektionsvorgang in der Form von Stoffzustand und/oder Stoffbestand an bestimmten Stoffen enthaltenen Informationen kann der Fachmann - unter der Voraussetzung ihrer fachgerechten Aufbewahrung - auch nach beliebig langer Zeit mittels bekannter chemischer und/oder physikalischer Untersuchungsmethoden jederzeit mit an und für sich bekannten Analytikmitteln abgreifen.

Zusammengefaßt lassen sich das erfindungsgemäße Detektionsverfahren und die erfindungsgemäßen Detektoren damit zur angenäherten quantitativen Ermittlung des Stoffgehaltes einzelner oder mehrerer Stoffe in Flüssigkeiten, und unabhängig davon oder gleichzeitig zur Ermittlung von Richtungs-und Geschwindigkeits-Parametern von insbesondere wäßrigen Flüssigkeitsströmen einsetzen, indem der Detektor durch Stoffaufnahme und/oder Stoffabgabe und/oder durch Änderung der Wirkstoffzusammensetzung seinen Stoffbestand verändert, und zwar abhängig von a1) dem zu detektierenden Stoffbestand und/oder von der zu detektierenden Strömungsgeschwindigkeit, a2) der durch den Einflußbereich des Detektors geströmten Flüssigkeitsmenge und a3) der gewählten Zeitdauer des Detektionsvorganges.

Mit dem erfindungsgemäßen Verfahren werden somit Überwachungs-und Analysentechniken verfügbar, deren Anwendung sehr einfach ist und die lückenlos durchgeführt werden können. Dank kontinuierlicher Hindurchführung eines erfindungsgemäßen Detektionspapiers oder Detektionsfadens durch ein natürliches wäßriges Medium, durch Abwasser, Produkte, Rohstoffe und Zwischenprodukte können b1) lückenlose Rückstellmuster generiert und b2) lückenloses Detektor-Material für die Qualitätskontrolle gewonnen werden. Als Beispiele sind die Wasser- und Abwasseruntersuchung, die Weineueugung, Pommes-frites-Öl sowie die Bier- und Getränke-Produktion zu nennen.

Grundsätzlich eignen sich die erfindungsgemäßen Detektoren für alle Felder der Analytik und Probenahme einschließlich Probenkonservierung. So kann man die Detektoren nach dem Detektionsprozeß beliebigen Schritten an sich bekannter Probenbehandlungs-, Probenaufbewahrungs- und Analysenverfahren unterwerfen. Zur Probenkonservierung verwendet man vorzugsweise einen oder mehrere der Verfahrensschritte Trocknen, Zerteilen, Aufspulen, Aufspulen mit Trennfolie, Kühlen, Einfrieren und Unter-Inertgas-Setzen. Auch den Analysenverfahren sind keine Grenzen gesetzt. Die auf dem Detektor vorliegenden Proben können z.B. dem Veraschen, Extrahieren, Eluieren, Derivatisieren und beliebig langem Aufbewahren unterworfen werden.

Die Träger der erfindungsgemäßen Detektoren sind entweder hochgradig permeabel für die zu untersuchende Flüssigkeit oder sie sind derart gestaltet, daß sie der Strömung der Flüssigkeit keinen wesentlichen Widerstand entgegensetzen. Eine hydrodynamisch günstige Formgebung kann z.B. erreicht werden, indem die Detektoren als Streifen, Bänder, Faden oder Filterwolle eingesetzt werden, die ein hinreichend großes Zwickelvolumen enthalten.

Die neuartigen Detektoren sind derart konsistent, daß während des Einsetzens und Herausnehmens aus dem Untersuchungsmedium und während des Detektionsvorgangs kein wesentlicher Materialverlust durch mechanischen Abrieb und das Herausfallen oder Abbrechen von Teilchen entsteht.

Der Detektor ist bevorzugt mit einer Vorrichtung ausgestattet, die durch eine oder mehrere Funktionen des Zusammenhaltens, des Schutzes gegen Zerfall und gegen mechanische Zerstörung, der Formgebung, der Strömungsleitung, der Fixierung, der Auftriebvermittlung, der Abtriebvermittlung, der elektrischen Isolation und der Einhausung der Detektorbestandteile gekennzeichnet ist.

Für die linienförmige, flächenförmige oder volumenförmige Untersuchung von großräumigen wäßrigen Systemen werden als Form der Träger vorzugsweise Bänder, Streifen, Fäden und/oder Fasern, leere oder gefüllte Schläuche, Leisten, Drähte, leere oder gefüllte perforierte Rohre, leere oder gefüllte zylindrische oder andersartig geformte Rohre oder Hülsen, leere oder gefüllte längliche Körbe oder Netzschläuche, Stangen, Leisten usw. gewählt, also gemäß ihrem äußeren Erscheinungsbild in ihrer Topographie ähnliche Gebilde, bei denen nämlich eine Raumdimension gegenüber den beiden anderen dominiert. Die einzelnen Detektoren können aber auch andere Formen haben, z. B. solche, bei denen zwei Raumdimensionen gegenüber der dritten dominieren, wie z. B. Papierblätter, flache Taschen, Folien, Furniere, textile Gebilde oder Nonwovens. Insbesondere für die Untersuchung punktförmiger Kompartimente wären für die Untersuchung auch solche Detektoren geeignet, bei denen die drei Raumdimensionen von etwa ähnlicher Ausdehnung sind, z.B. sphärische Behälter, Beutel, Säcke, Hülsen (nicht Teil der beanspruchten Erfindung). Die Detektoren dieser Form werden hauptsächlich an einem Halteelement dicht untereinander bzw. übereinander angeordnet, wenn sie fur die linien-, flächen- oder volumenförmige Untersuchung vorgesehen sind. Ein solches Halteelement mit den daran angeordneten Detektoren kann im praktischen Betrieb wie ein länglicher Detektor betrachtet werden.

Die Einhausung des Detektors hat vorzugsweise eine oder mehrere der Eigenschaften: hohe bis geringe hydraulische Durchlässigkeit auf einer oder mehreren Seiten. Sie kann aber auch aus Erscheinungsformen wie Netz, Folie, Textil, Gewebe, Vlies, Papier, Nonwoven, perforiertes Rohr, Verbundmaterial, Gestrick, Drahtgitter, Lochblech bestehen und sogar selbst über Detektionseigenschaften verfügen.

Die Detektoren werden rückholbar in eine Grundwasser-, Abwasser- oder Oberflächenwasser-Meßstelle verbracht und zum Zweck der Untersuchung nach einer gewissen Zeit entnommen. Bevorzugt werden Kompartimente aus den Gruppen Abwasser, Grubenwasser, Deponie-Sickerwasser, Trinkwasser, Genußmittel, Getränke, industrielle Kühl- und Kreislaufwässer, Kernkraftwerk-Kreislaufwasser, -Abwasser, Waschwasser mit den Detektoren untersucht.

Der Träger kann während des Detektionsvorgangs auch als Dispersion von faserförmigen bzw. Fasern enthaltenden Teilchen vorliegen. Die Detektoren enthalten vorzugsweise einen Anteil von einem oder mehreren Wirkstoffen zur Detektion, der sorptionsaktiv und/oder reaktiv auf mindestens einen zu untersuchenden Bestandteil der Lösung reagiert und/oder einen Anteil von einem oder mehreren Wirkstoffen zur Detektion, der adhäsiv und/oder reaktiv auf mindestens einen zu untersuchenden Bestandteil der Lösung reagiert und/oder einen Anteil von einem oder mehreren Wirkstoffen, die feldaktivierbar sind und/oder die dem Detektor mittels Federspannung eine Spannung aufprägen.

Dabei kann der Wirkstoff zur Detektion in Form einer Beschichtung als Partikel und/oder als Faser vorliegen und der Wirkstoffinhalt kann mindestens eines der nachfolgend aufgeführten Merkmale A), B) oder C) aufweisen, und der oder die gegebenenfalls vorhandenen Hilfsstoffe können mindestens eines der Merkmale D) und/oder E) aufweisen und die Konsistenz des Detektors kann mindestens eines der Merkmale F) bis L) aufweisen:
A) mindestens ein Wirkstoff zur Detektion ist in der Form einer Beschichtung und/oder Imprägnierung in definiert inhomogener Verteilung in dem Fasermaterial derart angeordnet, daß der Wirkstoff in einem vorbestimmten Anteil der das Fasermaterial bildenden Fasern und/oder Partikeln in einer vorbestimmten Menge vorliegt, und der Wirkstoff ist in einem weiteren vorbestimmten Anteil der das Fasermaterial bildenden Fasern und/oder Partikel nicht oder in einer vom ersten vorbestimmten Anteil der das Fasermaterial bildenden Fasern und/oder Partikeln abweichenden Menge vorhanden;
B) in dem Detektor sind ein oder mehrere der Wirkstoffe zur Detektion in definiert homogener Verteilung in einer oder mehrerer der Formen: Fasern, Partikel, Imprägnierung und/oder Beschichtung von Fasern und/oder Partikeln enthalten;
C) feldaktivierbare Hilfsstoffe sind mittels adhäsiv wirkender Hilfsstoffe in und/oder an dem Detektor gebunden, und das Fasermaterial enthält gegebenenfalls einen oder mehrere Wirkstoffe zur Detektion;
D) feldaktivierbare Hilfsstoffe sind durch mindestens eine der Feldwirkungen: Gravitationsfeld, Fliehkraftfeld, Magnetfeld und elektrisches Feld aktivierbar;
E) der Detektor wird dem zu untersuchenden Kompartiment in einer oder mehrerer der Konsistenzklassen zugegeben: fester Faserverbund gegebenenfalls mit Partikelanteil, feste Klebefolie gegebenenfalls mit Faser-und/oder Partikelanteil, Faserbrei gegebenenfalls mit Partikelanteil;
F) der Detektor liegt in dem zu untersuchenden Kompartiment in einer oder mehrerer der Konsistenzklassen vor: fester Faserverbund gegebenenfalls mit Partikelanteil, feste Klebefolie gegebenenfalls mit Faser-und/oder Partikelanteil, Faserbrei gegebenenfalls mit Partikelanteil;
G) der Detektor liegt nach der Detektion in dem zu untersuchenden Kompartiment in einer oder mehrerer der Konsistenzklassen vor: loser bis fester Faserverbund gegebenenfalls mit Partikelanteil, feste Klebefolie mit Partikelanteil und gegebenenfalls mit Faseranteil;
H) der Detektor liegt nach der Detektion in dem zu untersuchenden Kompartiment in einem oder mehreren der Zustände vor: getrockneter und/oder eingefrorener loser bis fester Faserverbund gegebenenfalls mit Partikelanteil, getrocknete und/oder eingefrorene feste Klebefolie mit Partikelanteil und gegebenenfalls mit Faseranteil;
I) der Detektionsvorgang wird durch die künstliche Einwirkung von Hilfsstoffen, wie Reduktionsmitteln oder Oxidationsmitteln unterstützt und/oder gelenkt;
J) der Detektor enthält einen oder mehrere Schutzstoffe gegen Mikroben;
K) der Detektor wird innerhalb des Untersuchungsmediums von diesem passiv durchströmt.

Vorzugsweise enthalten die Detektoren Fasern, worunter hier neben den eigentlichen Fasern auch alle solche Gebilde verstanden werden, deren makroskopisches äußeres topologisches Erscheinungsbild demjenigen von länglichen Partikeln oder Gegenständen entspricht. Bei länglichen Gegenständen ist eine der Raumdimensionen dominant gegenüber den beiden anderen Raumdimensionen. Deshalb werden hier auch zu den Fasern Gegenstände wie folgende gezählt: beispielsweise Stäbe, Drähte, Leisten, Metallwollen, Stahlwolle, Papierstreifen, Krepppapierstreifen, Zellstoffstreifen, Papierwolle - damit ist zu schmalen Streifen zerschnittenes Papier gemeint -, Textil-, Vlies- und sonstige Nonwovenwolle - damit sind zu schmalen Streifen zerschnittene Textil-, Vlies-und sonstige Nonwovenbahnen gemeint -, Textilstreifen, Folienbänder, Folienwolle - damit ist zu schmalen Streifen zerschnittene Folienbahn gemeint -, Kunststoffbänder, Fäden oder auch Röhren und Hohlfasern. Vorzugsweise sind die Wollen, Fasern Streifen und Bänder in hydraulisch leicht durchlässiges Material eingehaust. Dies sind vorzugsweise Schläuche aus Geweben, Vliesen, Tell, Gestricken und Netzen. Von den eingesetzten Detektoren ist vor ihrem Einsatz vorzugsweise bekannt: Gewicht, Wassergehalt, Meßstoffgehalt, Wirkstoffgehaft und Wirkstoffzusammensetzung, um die nach der Probenahme eingetretenen Veränderungen quantitativ auswerten zu können.

Aus der Gruppe der Fasern werden folgende Typen bevorzugt: organische Naturfasern, anorganische Naturfasern, organische Kunstfasern, anorganische Kunstfasern, chemisch modifizierte Fasern natürlicher Herkunft, physikalisch modifizierte Fasern natürlicher Herkunft, chemisch modifizierte Fasern künstlicher Herkunft, physikalisch modifizierte Fasern künstlicher Herkunft, unverrottbare Fasern, schwer verrottbare Fasern, mit Jod stabilisierte organische Fasern, mit Silber stabilisierte organische Fasern.

Die Träger werden in einer oder in einer oder mehrerer der folgenden Formen eingesetzt: Papiere, Papierwollen, Folien, Folienwollen, Metallwollen, Metallfasern, zur Kräuselung einseitig gereckte Wollen, Gewebe, Gewebewollen, Schaumstoffwollen, Streifen, Fasern, Fäden, Drähte, Gestricke, Metallfolien, Folien, Nonwovens, textile Gebilde, Gewebe, Vliese, Späne, Drehspäne, Metallspäne, Metallgewebe, Nichtmetallgewebe, Nonwovens, Textilien, Fasern, Faden, Zellstoffe, Vliese, Netze, Gestricke, Metallgestricke.

Vorzugsweise enthalten sie zumindest Bestandteile von einem oder mehreren der folgenden Stoffe, deren Oberfläche durch Fältelung, Prägung, Einstiche, Lochungen, Kreppen vergrößert wurde: Papier, Papierwollen, Folien, Folienwollen, Metallfolien, Metallwollen, Gewebe, Gewebewollen, Schaumstoffwollen, Streifen, zur Kräuselung einseitig gereckte Folienwollen, Vliese, Metallgewebe, Nichtmetallgewebe, Nonwovens, Textil.

Für bestimmte Anwendungszwecke, zum Beispiel im Langzeiteinsatz, enthält der Detektor vorzugsweise eine oder mehrere Formen künstlich hergestellter Fasern und/oder künstlich hergestellte Fasern, die einer modifizierenden chemischen und/oder physikalischen Behandlung unterworfen worden sind, aus den Gruppen der Keramikfasern, Kohlenstofffasern, Aktivkohlefasern, Glasfasern, Mikroglasfasem, Kunstharzfasern, Mikroolefinfasern, Metallfasern.

Besonders bevorzugt enthält er mindestens einen Bestandteil aus einem oder mehreren der folgenden Stoffe, deren Oberfläche durch Zick-Zack-Schnitt oder Wellschnitt, Fältelung, Prägung, Einstiche, Lochungen, Kreppen vergrößert wurde: Papier, Papierwollen, Folien, Folienwollen, Metallfolien, Metallwollen, Gewebe, Gewebewollen, Schaumstoff, Schaumstoffwollen, Streifen, zur Kräuselung einseitig gereckte Folienwollen, Vliese, Bleche, Furniere, Metallgewebe, Nichtmetallgewebe, Nonwovens, Textil.

Vorzugsweise enthält der Detektor mindestens einen der Bestandteile: Naturfasern, Naturfasern, die einer modifizierenden Behandlung unterworfen worden sind, künstliche Fasern, Zellstoffe, Halbzellstoffe, Strohzellstoffe, Reisstrohzellstoffe, Bambuszellstoffe und Bambusfasern, Baumwollzellstoffe, Espartozellstoffe, Bagassezellstoffe, Zellstoffe enthaltend Fasern aus Sisal, Flachs, Nessel, Baumwolle, Jute, Ramie, Hanf, Hochausbeutezellstoffe, Bisulfitzellstoffe, Neutralsulfit-Strohstoffe, Holzschliffe, gebleichte Holzschliffe, Braunschliffe, chemische Holzschliffe, Weißschliffe, Defibratorschliffe, Libby, Calciumalginatfaser-haltige Zellstoffe oder auch im Recycling aus Papieren, Pappen, Zellstoffen, Textilien und Papiersäcken gewonnene Faserstoffe. Bevorzugt sind aber auch Detektorbestandteile aus einer oder mehrerer Gruppen der Keramikfasern, Kohlenstofffasern, Aktivkohlefasern, Glasfasern, Mikroglasfasern, Kunstharzfasem, Mikroolefinfasem, Metallfasern. Diese können ebenfalls einer chemischen Behandlung zur Modifikation unterworfen worden sein.

Vorzugsweise stammt das in den Detektoren enthaltene Fasermaterial in der Form von Papier und/oder wirkstoffhaltigern Zellstoff aus der Papier- oder Zellstoffproduktion oder auch außerhalb von beiden Produktionen, wobei dessen Wirkstoffgehalt entweder komplett oder teilweise in der Zellstoffproduktion in das Fasermaterial integriert wurde oder er komplett oder teilweise außerhalb der Zellstoffproduktion und außerhalb der Papierproduktion oder komplett oder teilweise in der Papierproduktion integriert wurde.

Bevorzugtes Material fur die Träger ist Wirkstoffe enthaltende Wolle aus Papier. Die Herstellung der Wolle aus Papier kann in an und für sich bekannten Einrichtungen geschehen, mit denen Papierbahnen in schmale Streifen geschnitten werden. Mittels derartiger Einrichtungen können auch Papierwollstreifen hergestellt werden, deren einzelne Papierstreifen nicht gerade geschnitten sind, sondern die zickzack- oder wellenförmig geschnitten sind. Bevorzugt ist auch ein nicht schneidendes Trennen des Papiers, was den Vorzug wirksamer Vergrößerung der Stoffaustausch-Oberfläche hat. Die bevorzugte Breite der einzelnen Papierstreifen, aus denen sich die erfindungsgemäße Wolle zusammensetzt, liegt zwischen 0,01 mm und 1 cm; besonders bevorzugt ist eine Breite der Papierstreifen zwischen 0,1 mm und 2 mm.

Wolle aus Papier mit sehr schmalen Papierstreifen wird bevorzugt aus sehr dünnem Papier hergestellt; solche aus breiteren Papierstreifen bevorzugt aus entsprechend dickerem Papier. Bevorzugt sind Papiere mit Flächengewichten zwischen 10 g/m² und 200 g/m². Die Wolle aus Papier wird bevorzugt aus solchen Papieren hergestellt, die eine oder mehrere solcher Wirkstoffkomponenten enthalten, die geeignet sind, den beabsichtigten Hauptzweck des Papierwolle-Einsatzes, nämlich den Detektionsvorgang, möglichst effektiv zu gestalten. Es ist aber ohne Weiteres möglich, eine oder mehrere Wirkstoffkomponenten nach ihrer Herstellung auf die Wolle aus Papier zu applizieren oder auch im Verlaufe ihres Einsatzes als Detektor abzuscheiden. Es hat sich gezeigt, daß die erfindungsgemäßen Detektoren aus Papierwolle hervorragende Detektor-Eigenschaften haben.

Für kurzfristigen Einsatz im Wasserbereich und zur Probenahme in nichtwäßrigen Flüssigkeiten eignen sich besonders die organischen Naturfasern. Durch Einsatz von Jod- und/oder Silberverbindungen lassen sich organische Faserkomponenten und Wirkstoffe in der Wolle aus Papier ebenfalls gegen Verrottung bzw. Verkeimung stabilisieren.

Es kann vorteilhaft sein, die Träger aus Papier nicht als Endlos-Papierstreifen einzusetzen, sondern aus Papierstreifen, die vorzugsweise auf Längen von unter 200 mm, besonders bevorzugt auf Längen von unter 100 mm geschnitten sind, weil sie sich dadurch mit besserer Verteilung im Detektor einbauen lassen. Detektoren müssen nicht notwendig aus einer Vielzahl von Papierstreifen als Wolle bestehen. Auch einzelne Papierstreifen können als Träger in Detektoren eingesetzt werden. Einzelne oder mehrere gebündelte Papierstreifen oder lockere Stränge aus Papierstreifen, z.B. in einer Papierstreifenbreite, wie sie für Luftschlangen üblich ist und breiter, lassen sich sehr gut auch in beliebiger Lange als Träger in Detektoren einsetzen. Vorzugsweise werden auch sie in einem Netzschlauch eingehaust, um ihnen eine günstige Stabilität zu geben.

Bei Bedarf kann auch Wolle for die Detektoren aus multifunktionalen Papieren hergestellt werden. Es ist aber auch möglich, für diesen Zweck Papierwollen mit entsprechenden Einzel-oder Mehrzweckfunktionen zu mischen, um das gewünschte Detektionsergebnis zu erzielen.

Es hat sich herausgestellt, daß insbesondere die als Bahnen, Streifen, Konfetti, Schnipsel oder Wolle eingesetzten Detektormaterialien in eine wesentlich wirksamere Form gebracht werden können, wenn sie eine zusätzliche Formgebung erhalten. Durch diese erfindungsgemäße Formgebung erhalten diese zellstoff-, papier- und folienwolleartigen Filtermaterialien eine wellen- oder zickzackförmige Fältelung. So lassen sich aus dem Filtermaterial Detektoren herstellen, die sich durch eine wesentlich gleichförmigere hydraulische Durchströmung auszeichnen, als es mit dem ungefältelten Material möglich ist, das nämlich zu stark inhomogener Durchströmung neigt.

Insbesondere mit gefälteltern wolleartigen Filtermaterial hergestellte längliche Trägermaterialien, z.B. mittels Kunststoffnetz zu schlauch- oder wurstformig gefertigte Detektoren, haben sehr gute Eigenschaften, weil sie durch die gleichförmige Durchströmung sehr homogen beladen oder zur Reaktion gebracht werden können.

Vorzugsweise geschieht die Wellen-oder Zickzack-Prägung durch ein gegenläufiges Walzenpaar, das auf seiner zylindrischen Oberfläche achsenparallele Nuten der entsprechenden Formung hat, die bei der gegenläufigen Drehung der beiden Walzen zahnradförmig ineinandergreifen. Vorzugsweise nach Fertigung der Wolle, Streifen, Schnipsel oder Konfetti werden diese zwischen die aufeinander zulaufenden Zähne des gegenläufig drehenden Walzenpaars eingeführt und können nach Durchlaufen des Walzenpaarspalts in der gefältelten Form gewonnen werden.

Die durch Schneiden von Papier und Zellstoffen hergestellten Wollen aus schmalen Papierstreifen können um noch eine weitere Stufe für den hier beschriebenen Anwendungszweck verbessert werden. Dies geschieht, indem die Papiere derart zu Papierstreifen aufgetrennt werden, daß das Papier beim Trennvorgang ganz oder zumindest teilweise nicht glatt durchgeschnitten wird, sondern abgeschert oder gerissen ist. Dadurch enthalten die entstandenen einzelnen Streifen, aus denen die Wollen bestehen, zahlreiche Fasern, die aus den Streifen herausragen. Die derart hergestellten Wollen haben eine höhere Aktivität als die Wollen aus Streifen mit glatten Schnitträndern.

Als suspendierte Träger Detektoren werden solche Materialien vorgezogen, die sich besonders leicht aus der wäßrigen Phase abtrennen lassen. Dazu gehören z.B. alle Arten von Fasern, Drahtabschnitte, Konfetti aus Papieren, Zellstoffen, Folien, Textilien, Nonwovens, ferner Häcksel von Wollen aus Metall, Papier, Folien, Vlies, Textilien und Nonwovens.

Nach dem erfindungsgemäßen Verfahren kann es aber auch vorteilhaft sein, den Detektor-Fasergebilden durch eine oder mehrere der Feldwirkungen: der Gravitation, des Magnetismus, der Elektrizität oder der übertragenen Einwirkung von Feder- oder Gummispannung oder zusätzlich durch Reibung des bewegten Fluids an den faserhaltigen Materialien neben der Spannung auch ein Bewegungsmuster aufzuprägen.

Als für das Verfahren geeignete Haltevorrichtungen für Detektoren können neben länglichen Haltern, z.B. Stäben, Drähten, Meßbändern, Kordeln, Angelschnüren auch Vorrichtungen dienen, die auf der zu untersuchenden Flüssigkeit schwimmen. Diese Haltevorrichtungen können unterschiedliche Formen und Eigenschaften haben. Besonders bevorzugt sind quer zur Wasserströmung ausgerichtete längliche Haltevorrichtungen als Schwimmkörper, z.B. Hohlkörper oder Holzbalken.

Die Änderung im Stoffbestand und/oder Stoffzustand der Detektoren während des Detektionsprozesses am Detektionsort geschieht nach an und für sich bekannten Prinzipien der chemischen und physikalischen Reaktionsabläufe. Die derart gemäß den zu untersuchenden Parameter und Zielsubstanzen typisch veränderten Detektoren können danach einem geeigneten Transport sowie einer Lagerung oder Zwischenlagerung unterzogen werden. Falls es erforderlich ist, können sie zu einem späteren Zeitpunkt, z.B. zum Zweck der Reklamationsabwendung, unterschiedlichen physikalischen, chemischen oder sensorischen Untersuchungen unterzogen werden, um den untersuchten Zustand an der punkt-, linien-, flächen- oder raumförmigen Untersuchungslokalität zu einem oder mehreren Zeitpunkten oder während eines ununterbrochenen Zeitabschnittes abzubilden.

Die Detektoren enthalten einen Anteil von einem oder mehreren Wirkstoffen zur Detektion, die unter Detektionsbedingungen zur Stoffdetektion über mindestens eine der folgenden Eigenschaften verfügen, bezogen auf das zu detektierende Stoffinventar im Untersuchungs-Kompartiment: adhäsiv, adsorptiv, absorptiv, chemisch reaktiv, katalytisch aktiv, photokatalytisch aktivierbar, aktivierbar durch Aufprägen eines elektrischen Feldes, und/oder die zur Strömungsparameterdetektion über die Eigenschaft, Absonderung gelöster Reaktionsprodukte verfügen.

Auf Grund dieser Eigenschaften verändern die Detektoren ihre Zusammensetzung mindestens in Abhängigkeit von den Parametern: Konzentration der zu messenden Fluidinhaltsstoffe, und/oder in Abhängigkeit von der durchschnittlichen Fluid-Strömungsgeschwindigkeit an den Detektoren und der Zeitdauer des Kontaktes zwischen dem zu messenden Fluid mit den Detektoren des zu messenden Fluids. Die Masse des im Detektor vorliegenden Wirkstoffinventars kann als Folge der Reaktion mit dem zu detektierenden Stoffinventar während des Detektionsvorgangs abnehmen oder zunehmen, jedoch nur selten gleichbleiben.

Die erfindungsgemäßen Detektoren sind derart zusammengesetzt, daß sie in dem zu untersuchenden Medium mit einer oder mehreren zu detektierenden Substanzen die gewünschte(n) Reaktion(en) eingehen und diese in der Form einer Änderung ihres Stoffbestandes und/oder Stoffzustandes in ausreichender Menge derart speichern können, daß die uneingeschränkte Detektionswirksamkeit über den gewählten Meßzeitraum aufrechterhalten wird. Sie enthalten in dazu genügendem Vorrat einen oder mehrere Wirkstoffe, mittels derer die gewünschte Reaktion ausgelöst werden kann. Dabei können die Detektoren als solche aus Wirkstoffen bestehen oder den Wirkstoff als Imprägnierung oder Beschichtung, als chemisch gebundene Wirkstofffunktionen, als gebundene wirkstoffhaltige Partikel oder als unterschiedliche Wirkstoffe enthalten. Bereits mit wenigen Wirkstofftypen, die in den Detektoren enthalten sind, lassen sich zahlreiche Klassen von Stoffen aus Flüssigkeiten detektieren.

Beispiele für Stoffe und dafür geeignete Detektions-Wirkstoffe sind:
- suspendierte Feststoffe, die an mit körnigen und/oder faserhaltigen Materialien und vorzugsweise mit organischen und/oder anorganischen Gelen beschichteten Detektoren gebunden werden können,
- suspendierte Öle, die an vorzugsweise an mit hydrophoben Mitteln beschichteten oder daraus bestehenden körnigen und/oder faserhaltigen Detektoren gebunden werden können,
- Schwermetalle, Radionuklide, Aluminium, Arsenide, Sulfide, reduzierte Schwefelverbindungen, polare Aromaten, Cyanide, Selenide, die an vorzugsweise mit Oxiden und Mischoxiden des Eisens und/oder Mangans und/oder metallisches Eisen enthaltenden Partikeln beschichteten oder daraus bestehenden körnigen und/oder faserhaltigen Detektoren gebunden werden können,
- Schwermetalle, Aluminium, Erdalkalien, die an vorzugsweise mit Carboxylsäure- bzw. Carboxylat-Gruppen enthaltenden Stoffe gebunden werden können, wie z.B. Seifen, Fettsäuren, Kalkseifen, Huminstoffen, Huminatsalzen, Alginaten, mit denen Sorbenten-Desorbenten-Partikel oder -Fasern beschichtet sind oder aus denen diese bestehen, körnige und/oder faserhaltige Detektoren,
- Baryt, Eisen-und Mangan-Oxide, die durch reduzierende Stoffe aufgelöst werden können,
- Bromide, Jodide, Chloride und organische Verbindungen, die an vorzugsweise mit Aktivkohlepartikeln beschichteten oder daraus bestehenden Detektoren gebunden oder umgesetzt werden können,
- Jod, das an vorzugsweise mit Stärke beschichteten oder daraus bestehenden Detektoren gebunden oder umgesetzt werden kann,
- Fluorid, das an vorzugsweise mit mindestens einem Stoff aus den Gruppen der mit Aluminiumhydroxiden und Eisen-III-hydroxiden beschichteten oder daraus bestehenden Detektoren gebunden werden kann,
- Quecksilber, das an vorzugsweise mit mindestens einem Stoff aus den Gruppen der Ionenaustauscher oder amalgamierbaren Metallen beschichteten oder daraus bestehenden Detektoren gebunden werden kann,
- Erdalkalien, die an vorzugsweise mit mindestens einem Stoff aus den Gruppen der Ionenaustauscher, Alginsäuren, Zeolithe, Huminstoffe beschichteten oder daraus bestehenden Detektoren gebunden werden können,
- lipophile Substanzen, die an vorzugsweise mit mindestens einem Stoff aus der Gruppe der Aktivkohlen, Polyolefine, porösen und ölbeschichteten Polyolefine, Polyolefinfasern, Wachsen, wachsbeschichteten Fasern, Aktivkohlefasern, aktivkohlebeschichteten Fasern gebunden werden können.

Die Untersuchungen, die zu der Erfindung geführt haben, haben auch gezeigt, daß sich die schwerlöslichen Erdalkalisalze einschließlich ihrer Derivate wie freie Saccharide hervorragend als Detektoren für gelöste Schwermetalle eignen und damit ideal in das erfindungsgemäße Verfahren integrieren lassen.

Besonders geeignet dafür sind die Salze des Polysacharid-Naturproduktes Alginsäure, weil sich dieses Polysaccharid in großen Mengen aus Algen gewinnen läßt und damit zu den nachwachsenden Rohstoffen zählt. Bevorzugt ist dies dann der Fall, wenn die Alginsäuren und ihre Derivate als freie Alginsäuren oder als Erdalkalisalze zum Einsatz gelangen. Dabei wird vorzugsweise nach folgenden VerfahrensVarianten vorgegangen:
I) Alginsäure liegt als Beschichtung vor auf einem oder mehreren der Feststoffe: Partikel, Fasern, Faden, Papieren, Vliesen, Geweben oder sonstigen vorzugsweise oberflächenreichen Körpern, die in der Detektor-Vorrichtung als Reaktivstoff wirken. Die gelösten Schwermetalle werden in diesem Fall in der Detektor-Vorrichtung durch die Alginsäure fixiert. Die Beschichtung der oberflächenreichen Körper geschieht vorzugsweise durch Tränken mit löslichem Alginat, das anschließend mit Säure als Alginsäure ausgefällt wird.
II) Vorzugsweise feinteilige Alginsäurepartikel werden mit einem Bindemittel, bestehend vorzugsweise aus einem fällbaren Polysaccharid - besonders bevorzugt dafür sind die Alkalisalze der Alginsäure -, auf einem oder mehreren der Feststoffe fixiert: Partikel, Fasern, Fäden, Papiere, Vliese, Gewebe oder sonstige vorzugsweise oberflächenreiche Körper, die in der Detektor-Vorrichtung als Reaktivstoff wirken. Die gelösten Schwermetalle werden in diesem Fall in der Detektor-Vorrichtung durch die Alginsäurepartikel fixiert. Die Beschichtung der oberflächenreichen Körper mit Bindemittel geschieht vorzugsweise durch Tränken mit löslichem Alginat, das anschließend mit Säure als Alginsäure in die Bindemittelphase überfuhrt wird.
III) Calcium-, Strontium-, oder Bariumalginat liegt als Beschichtung vor auf einem oder mehreren der Feststoffe: Partikel, Fasern, Fäden, Papiere, Vliese, Gewebe oder sonstige vorzugsweise oberflächenreiche Körper, die in der Detektor-Vorrichtung als Reaktivstoff wirken. Die Beschichtung der oberflächenreichen Körper geschieht vorzugsweise durch Tränken mit löslichem Alginat, das anschließend mit mit löslichen Calciumsalzen als Calciumalginat ausgefällt wird. Die gelösten Schwermetalle werden in diesem Fall in der Detektor-Vorrichtung durch die schwerlöslichen unter Austausch von Erdalkali-Ionen in die gelöste Phase fixiert.
IV) Vorzugsweise feinteilige Erdalkalialginatpartikel werden mit einem Bindemittel, bestehend vorzugsweise aus einem fällbaren Polysaccharid - besonders bevorzugt dafür sind die Alkalisalze der Alginsäure - auf einem oder mehreren der Feststoffe fixiert: Partikel, Fasern, Fäden, Papiere, Vliese, Gewebe oder sonstige vorzugsweise oberflächenreiche Körper, die in der Detektor-Vorrichtung als Reaktivstoff wirken. Die gelösten Schwermetalle werden in diesem Fall in der Detektor-Vorrichtung durch die Alginatpartikel fixiert. Die Beschichtung der oberflächenreichen Körper mit Bindemittel geschieht vorzugsweise durch Tränken mit löslichem Alginat, das anschließend mit Erdalkalisalzen zu Erdalkalialginat als Bindemittelphase und/oder mit Säure zu Alginsäure als Bindemittelphase überführt wird.
V) Calcium-, Strontium-, oder Bariumalginat liegt in Faserform vor in Form von Einzel-Fasern, Fäden, Papieren, Vliesen, Gewebe oder sonstigem faserhaltigem Material, das in der Detektor-Vorrichtung als Reaktivstoff wirkt. Aluminium und die gelösten Schwermetalle werden in diesem Fall in der Detektor-Vorrichtung durch die schwerlöslichen Salze unter Austausch gegen die Erdalkali-Ionen fixiert.

Die Experimente fur die vorliegende Erfindung haben auch gezeigt, daß sich die aus in Wasser gelösten Carboxylat-Funktionen enthaltenden Polysaccharide, vorzugsweise löslichen Alginaten, gefällten freien Säuren, vorzugsweise Alginsäure oder ihre schwerlöslichen Salze, vorzugsweise die schwerlöslichen Salze der Alginsäure mit mehrwertigen Metallionen, hervorragend als Bindemittel dazu eignen, Fest-Reagens-Partikel an Fasern und Partikeln der Detektoren hinreichend fest zu fixieren, während die schwerlöslichen Erdalkalisalze der Carboxylat-Funktionen enthaltenden Polysaccharide zusätzlich in der Lage sind, in Wasser gelöste Schwermetalle und Aluminium zu sorbieren.

Der Beschichtungsvorgang zur Abscheidung von Wirkstoffen auf faserförmigen oder partikulären oder flächenförmigen Trägern gelingt bei alien Wirkstoffen, die sich als Produkte einer chemischen Reaktion zwischen mindestens zwei Reaktionsteilnehmern und/oder einer physikalischen Reaktion aus einem Medium niederschlagen lassen, das mindestens in einem der Materiezustände vorliegt aus den Gruppen: flüssige homogene Phasen, flüssige Emulsionen und Suspensionen, dichte Gasphasen, einfachen Gasphasen, feste Phasen. Dazu gehören insbesondere Stoffe wie
- organische Substanzen, darunter bevorzugt die Naturstoffe und ihre chemischen Modifikationen,
- natürliche und künstliche Huminstoffe und ihre Salze,
- in Wasser schwerlösliche oder unlösliche organische Säuren, z.B. Öl- und Fettsäuren und ihre Salze,
- Polysaccharide und ihre Derivate einschließlich ihrer Salze,
- Polynucleotide und ihre Salze,
- Kartoffel-Mais-und Reisstärke,
- Nucleinsäuren und ihre salzartigen Verbindungen,
- Lignine, Ligninabbauprodukte, Ligninsulfonsäuren sowie Salze der genannten Lignine,
- Gerbstoffe und ihre Salze,
- organische und anorganische Ionenaustauscher,
- Aktivkohle als Partikel oder Fasern, Ruß, und Fullerene, vorzugsweise als offene tubulare Fullerene,
- mit Stickstoff und/oder Schwermetallen aktivierte Kohlen und Ruße,
- mit Schwermetalloxiden pyrolyseaktivierte Kohlen, Zucker, Peche und Ruße, derivatisierte Aktivkohlen und Ruße,
- Acrylate; Zeolithe und Schichtsilikate,
- lipophile und oleophile Stoffe, insbesondere Wachse, Öle, Metallseifen,
- anorganische Sorbentien aus den Gruppen der Oxide, Sulfide, Selenide,
- anorganische Sorbentien aus den Gruppen der Schichtsilikate und Zeolithe, amalgamierbare Metalle,
- zur Aufnahme von Wasserstoff fähige Platinmetalle,
- organische Komplexbildner für anorganische Stoffe,
- Silber und Silbersalze,
- Platinmetalle und ihre Hydride,
- Platinmetalllegierungen und ihre Hydride,
- amalgamierbare Metalle, insbesondere Gold, Silber, Kupfer, Zink, Gold als Metallisierung, Kolloid und Blattgold,
- Lanthanide, Actinide, ihre Salze und Verbindungen wie z. B. Ceralkandionate,
- Schwermetalle, ihre Salze und Verbindungen, -Porphine und ihre Verbindungen,
- schwerlösliche Sulfide, Hydroxide, Oxide und Mischoxide als solche und als Korrosionsschichten auf Metalle, insbesondere diejenigen der Metalle Eisen, Nickel, Kupfer, Mangan, Zink, Lanthaniden, Actiniden, Scandium und Yttrium, Flockmittel, quellfähige Polymere und Polyelektrolyte,
- Alginsäure, Alginate und Chitosane,
- photolytisch aktivierbare organische und anorganische Stoffe,
- galvanisch, photochemisch oder chemisch gefällte Metallisierungen.

Viele der Wirkstoffe, die für das erfindungsgemäße Verfahren geeignet sind, werden bereits in WO-A-00/16877 sowie in DE-A-198 34 916 beschrieben. Diese Wirkstoffe werden in den Detektoren vorzugsweise eingesetzt als wirkstoffimprägnierte oder - beschichtete Fasern und/oder Partikel oder solche Fasern und/oder Partikel, die ganz oder zum Teil aus Wirkstoff bestehen. Darüber hinaus eignen sich weitere Wirkstoffe für die Detektoren, die in den vorliegenden Schriften genannt sind. Die in der folgenden Aufzählung aufgeführten schwerlöslichen Wirkstoffe eignen sich ebenfalls für das erfindungsgemäße Verfahren:
- mit Metalloxiden und Metall-Mischoxiden beschichtete Glimmerschuppen, Glasflakes, Glasbläschen und Cellulosefasern;
- alle in Wasser schwerlöslichen Oxide und Hydroxide,
- alle in Wasser schwerlöslichen Sulfide und Hydrogensulfide, organische Schwefelverbindungen, in Wasser schwerlöslichen Sulfate;
- in Wasser schwerlösliche Halogenide, in Wasser schwerlösliche Carbonate,
- in Wasser schwerlösliche Carboxylsäurederivate und ihre Salze,
- in Wasser schwerlösliche Alkandionate und ihre Schwermetall-Komplexverbindungen,
- in Wasser schwerlösliche organischen Salze oder Komplexverbindungen;
- Sulfide, Oxide, Mischoxide, Hydroxide, Dionate enthaltend eines oder mehrere Elemente der Lanthaniden und der Actiniden, Zirkon, Titan, Hafnium, Niob, Tantal, Zinn, Cer, Silicium, Aluminium, Nickel, Kupfer, Eisen, Mangan, Molybdän, Wolfram, Germanium;
- in Wasser schwerlösliche Polysaccharide und ihre Salze, Polyacrylate und ihre Derivate, Polyacrylsäuren und ihre Derivate, polymere Amine und ihre Salze, wasserunlösliche Huminstoffe, gelartige Naturstoffe, gelartige künstliche Stoffe, organische und anorganische Flockungsmittel, Alginate, Alginsäuren, Pektine, Chitosane, Chitosansalze, Xanthane, Xanthansalze, polymere Amine, Salze der polymeren Amine, organische Flockungsmittel, Huminstoffe und ihre salzartigen Verbindungen, aus denen durch eines oder mehrere Verfahren der Fällung, Flockung, Filtration die wasserlösliche Huminstofffraktion abgetrennt wurde;
- reduzierende Gase, oxidierende Gase, Inertgase, z.B. Wasserstoff, Sauerstoff, Ozon, Sauerstoffplasma, Peroxide, Magnesiumperoxid, Wasserstoffperoxid, Permanganat, Erdalkalihydroxide, Magnesiumhydroxid, Calciumhydroxid, Kaliumcarbonat, Natriumcarbonat, primäre, sekundäre und tertiäre Phosphate;
- Adhäsivstoffe, organische und anorganische Ionenaustauscher, Hydride, Zeolithe, ölaufnahmefähige Stoffe, feldaktivierbare Stoffe, Leichtstoffe, Schwerstoffe, Photokatalysatoren, elektrisch leitfähige Stoffe, magnetisierbare Stoffe, Metallhydride, Mikro-Hohlkugeln aus Kunststoff und Glas, Kork, Graphit, Ruß, Glimmer, Metalle, Metalllegierungen, Metallkolloide, galvanisch abgeschiedene Metalle, Zink, Eisen, photolytisch abgeschiedene Metalle, Polyolefinfasern, Glasfasern;
- Naturstoffe, ihre chemischen Modifikationen und salzartige Verbindungen davon, z.B. Pektine und ihre salzartigen Verbindungen, Pektinsäuren und ihre salzartigen Verbindungen, Protopektine und ihre salzartigen Verbindungen, Laminarane und ihre salzartigen Verbindungen, Lichenane und ihre salzartigen Verbindungen, Pustulane und ihre salzartigen Verbindungen, Amylosen und ihre salzartigen Verbindungen, Amylopektine und ihre salzartigen Verbindungen, Glykogene und ihre salzartigen Verbindungen, Dextrane und ihre salzartigen Verbindungen, Pullulane und ihre salzartigen Verbindungen, Nigerane und ihre salzartigen Verbindungen, Isolichenane und ihre salzartigen Verbindungen, Cyclodextrine und ihre salzartigen Verbindungen, Chitine und ihre salzartigen Verbindungen, Chitosane und ihre salzartigen Verbindungen, Dextrane und ihre salzartigen Verbindungen, Inuline und ihre salzartigen Verbindungen, Levane und ihre salzartigen Verbindungen, Xanthane und ihre salzartigen Verbindungen, Porphyrane und ihre salzartigen Verbindungen, Porphine und ihre salzartigen Verbindungen, Phthalocyanine und ihre salzartigen Verbindungen, Phleine und ihre salzartigen Verbindungen, Agarosen und ihre salzartigen Verbindungen, Carrageenane und ihre salzartigen Verbindungen, Arabinane und ihre salzartigen Verbindungen, Xylane und ihre salzartigen Verbindungen, Polyuronide und ihre salzartigen Verbindungen, Homopolysaccharide und ihre salzartigen Verbindungen, Galactane und ihre salzartigen Verbindungen, Heteropolysaccharide und ihre salzartigen Verbindungen, Glucane und ihre salzartigen Verbindungen, Glucuronane und ihre salzartigen Verbindungen, Galactomannane und ihre salzartigen Verbindungen, Glycanoxylane und ihre salzartigen Verbindungen, Arabinoxylane und ihre salzartigen Verbindungen, Glycanoglykuronane und ihre salzartigen Verbindungen, Tragacanthsäuren und ihre salzartigen Verbindungen, Mannane und ihre salzartigen Verbindungen, Fructane und ihre salzartigen Verbindungen, Arabinoxylane und ihre salzartigen Verbindungen, Glykane und ihre salzartigen Verbindungen, Isoglycane und ihre salzartigen Verbindungen, Proteoglykane und ihre salzartigen Verbindungen, Glykoproteine, Lipopolysaccharide, Heteropolysaccharide, saure Mucopolysaccharide und ihre salzartigen Verbindungen, Hyalouronsäuren und ihre salzartigen Verbindungen, Peptidoglykane und ihre salzartigen Verbindungen, Teichonsäuren und ihre salzartigen Verbindungen, Kapselpolysaccharide und ihre salzartigen Verbindungen.

Weitere Wirkstoffe sind: durch chemische Modifikation veränderte anorganische und organische Naturstoffe und durch chemische Modifikation veränderte anorganische und organische Kunststoffe, insbesondere durch kovalente Bindung dieser Stoffe an Funktionen wie Carboxylsäuren, Carboxylate, Amine, Ammoniumsalze, Alkylamine, Alkylamoniumsalze, Sulfonsäuren, Sulfate, Phosphonsäuren, Phosphonate, Phosphorsäureester, Phosphorsäureester-Salze, Thiole, Thiolate, Thiocarboxylsäuren, Thiocarboxylate, Dithiocarboxylsäuren, Dithiocarboxylate, Thiocarbamate, Dithiocarbamate, Dimercaptotriazine, Iminodiacetate, Kronenether, Aza- und Thiahomologe der Kronenether, Bipyridine, Cryptand-, Spherand-, und Podandfunktionen, Siderophorfunktionen, Cyclodextrine, Cryptophane, fullerenähnliche Coronanden-Käfige aus verbrückten Triazinringen und fullerenähnliche Käfigmoleküle gemäß DE-A-197 00 146, DE-A-197 11 078 und DE-A-44 32 045, Clathratbildner, z.B. Cryptophane, Trimesinsäurederivate, Triphenylmethanderivate, Perhydrotriphenylenderivate, Cyclophosphazenderivate, hexasubstituierte Benzole, Diacetylenderivate.

Insbesondere die Polysaccharide eignen sich zur Derivatisierung, weil sie gut haftende Beschichtungen mit hochwirksamen Kollektoreigenschaften auf den Fasern und Partikeln ergeben. Besonders bevorzugte Beispiele chemisch modifizierter Polysaccharide als Wirkstoffkomponenten für das erfindungsgemäße Verfahren sind: O-Carboxymethylcellulose, Alkylaminoalkylether der Polysaccharide O-(2-(Diethylaminoethyl)-Cellulose, (2-(Diethylaminoethyl)-Cellulose, Aminoalkylether der Polysaccharide, O-(2-(Diethylaminoethyl)-Derivate quervernetzter Dextrane, Reaktionsprodukte von Polysaccharid-Iminokohlensäurediestern mit Aminen, an Polysacchariden immobilisierte Enzyme, Ether und Thioether von Polysacchariden mit Thiolderivaten des Triazins, Ether von Polysacchariden mit Aminoderivaten des Triazins, Ether von Polysacchariden mit Carboxylsäurederivaten des Triazins.

Weitere Beispiele natürlicher Wirkstoffe sind Polynukleotide und ihre salzartigen Verbindungen, Nukleinsäuren und ihre salzartigen Verbindungen, Lignine, ihre Abbauprodukte und ihre salzartigen Verbindungen, Ligninsulfonsäuren und ihre salzartigen Verbindungen, Gerbstoffe und ihre salzartigen Verbindungen.

Weitere Wirkstoffe in Detektoren für das erfindungsgemäße Verfahren können sein: zur Bildung von Komplexen mit anorganischen Stoffen fähige organische Stoffe, Zeolithe, Aktiv-Kohlenstoff-Partikel, Aktiv-Kohlenstoff-Fasern, mit Stickstoff angereicherte Aktivkohlenstoffe, mit Carboxylatgruppen angereicherte Aktivkohlenstoffe, mit Carboxylsäuregruppen angereicherte Aktivkohlenstoffe, mit Sulfonatgruppen angereicherte Aktivkohlenstoffe, mit Sulfonsäuregruppen angereicherte Aktivkohlenstoffe, Fulleren-Kohlenstoffe und ihre Derivate, Hydride aus der Gruppe der Platinmetalle, mit Carboxylatgruppen angereicherte Öle, Wachse oder Harze, mit Carboxylsäuregruppen angereicherte Öle, Wachse oder Harze, überwiegend aliphatische hochsiedende Ole, immobilisierte Enzyme, Schichtsilikate, mit Silber, Palladium, Gold und anderen Edelmetallen beschichtete Aktivkohlefasern, Edelmetallkolloide, galvanisch und/oder photochemisch generierte Metallisierungen auf Trägem, schwerlösliche Verbindungen der Chalkogenide, schwerlösliche Verbindungen der Gruppe 3 des Periodensystems (Scandiumgruppe) mit Edelmetallen beschichtete Metalldrähte, unbeschichtete Metalldrähte, mit Huminstoffen, Seifen oder Carboxylsäuren beschichtete Keramikfasern.

Lipophile und oleophile Stoffe, z. B. Öle und Wachse, als Beschichtung auf potösen Polyolefinpartikeln, Partikel, Gewebe und Vliese aus Aktivkohle-, Glas- oder Metallfasern, Drähte und metallische Rundgestricke eignen sich aber ebenfalls als Detektoren. Insbesondere die manganhaltigen, Seltene-Erd-haltigen, titanhaltigen, eisenhaltigen und aluminiumhaltigen Oxidschichten, die durch Korrosion auf den metallischen Detektoren erzeugt werden können, eignen sich als Sorbens, Reagens, Katalysator und Photokatalysator für die Umsetzung anorganischer und organischer Komponenten mit physikalischen und chemischen Mitteln.

In den genannten Veröffentlichungen sind auch Partikel, Fasern und Folien beschrieben, die ebenfalls Bestandteil solcher Fasermaterialien sein können, die vollständig aus Wirkstoff bestehen. In zahlreichen Druckschriften und der Fachbuch-Literatur werden ebenfalls partikuläre und faserförmige Wirkstoffe beschrieben, die ganz und gar aus Wirkstoffkomponenten bestehen. Beispiele hierfür sind stickstoffhaltige Aktivkohlen, stickstoff und schwermetallhaltige Aktivkohlen, Aktivkohle-Fasern, Aktivkohlestaub, Zinkgranalien, Goldpartikel und -drähte sowie Blattgold, ionenaustauschende Pulverharze, sphärische Ionenenaustauscher-Partikel. Auch solche Stoffe sind als Wirkstoffe in den Detektoren geeignet.

Unter den eingesetzten Aktivkohlefasern oder Aktivkohlepartikeln sind jene aktiven Pyrolysate besonders bevorzugt, die unter der Einwirkung von Katalysatoren gebildet wurden, welche die Bildung von ein-oder mehrschaligen Kohlenstoff-Mikrofasern katalysieren, die auch unter dem Namen tubulare Fullerene oder Kohlenstoff-Whisker bekannt sind, und wobei der Pyrolyseprozeß bei Temperaturen im Bereich zwischen 500 °C und 1200 °C vorzugsweise zumindest teilweise in der Gegenwart reduzierender kohlenstoffhaltiger Gase geführt wurde. Zu diesen Katalysatoren zählen viele Schwermetalle, insbesondere das Eisen, aber auch Edelmetalle. Die unter diesen Bedingungen gebildeten Aktivkohlen zeichnen sich in der Regel dadurch aus, daß sie bereits im Pyrolyseprozeß aktiviert worden sind und allein zur Vergrößerung ihrer inneren Oberfläche nicht notwendig weiteren Aktivierungsschritten ausgesetzt werden müssen. Zu diesen Aktivkohlen zählen auch die entsprechenden eisenhaltigen Formstoffe, deren Herstellung in der DE-A-44 47 317 beschrieben wird.

Besonders bevorzugte Rohstoffe für das erfindungsgemäße Verfahren sind eisenhaltige Kohlenstoffvliese, deren Herstellungsprozeß auch den Prozeßschritt Behandlung mit reduzierenden Kohlenstoff enthaltenden Gasen unter einer Temperatur innerhalb des angegebenen Temperaturintervalls enthält. Die Träger, die als Fasern und/oder Partikel eingesetzt werden können, können aber auch aus Siliciumcarbid, hochschmelzendem Glas oder anderen temperaturbeständigen Stoffen bestehen. Die Dotierung der Aktivkohlen mit dem Katalysator kann durch Imprägnation geschehen.

Es ist aber auch möglich, den Katalysator bzw. den Katalysatorprecursor dispergiert oder gelöst im Faser-und/oder Partikelprecursor vor dem Faser- bzw. Partikel-Bildungsprozeß vorzulegen. Es können die bei der Pyrolyse entstehenden Kohlenstoff-enthaltenden Gase zur Aktivierung durch Fullerenbildung hinreichen, vorzugsweise wird aber der Pyrolyseprozeß zumindest zeitweise zusätzlich in der Gegenwart kohlenstoffhaltiger Gase durchgeführt. Nach der Pyrolyse können die aktiven, insbesondere fullerenhaltigen Pyrolyseprodukte weiter modifiziert werden, z.B. durch Gasphasen-Oxidation in der Gegenwart von Kaliumcarbonat, durch Sulfonierung, durch Blausäurebegasung bei erhöhter Temperatur, durch Ammoniakbegasung bei erhöhter Temperatur, durch Salpetersäure-Oxidation.

Besonders gut geeignet als Detektor sind auch die Pyrolyseprodukte, die erhalten werden aus eisen- oder andere kohlenstofflösende Metalle enthaltenden Fasermaterialien, wenn die Pyrolyse unter Kohlenstoff-Whisker-bildenden Reaktionsbedingungen geführt wurde. Die erhaltenen Pyrolyseprodukte können direkt oder auch nach physikalischer Behandlung (z.B. Zerkleinerung) und/oder chemischer Behandlung (z.B. Derivatisierung) als Wirkstoffe und/oder Wirkstoffträger in die erfindungsgemäßen Detektoren eingearbeitet werden.

Ein Papier, das sich hervorragend für die erfindungsgemäßen Methoden der Detektion von lipophilen Stoffen in wäßrigen Phasen eignet, enthält Polyolefinfasern, vorzugsweise aber solche mit Filamentdurchmessern von <10 µm. Derartige Fasern, die auch Mikrofasern genannt werden, können im Blas-Spinnverfahren hergestellt werden.

Grundsätzlich sind alle diejenigen Wirkstoffe als Bestandteile der Detektoren geeignet, die mit der Zielsubstanz, also der zu analysierenden Substanz, in direkter Reaktion oder nach vorausgehenden Reaktionen, wie sie z.B. durch die enzymgestützte oder photonenaktivierte oxidierende Mineralisation repräsentiert werden, im Detektor fixierte Reaktionsprodukte ergeben. Tabelle I gibt hierfür Beispiele an.

Grundsätzlich sind all jene Wirkstoffkomponenten für die erfindungsgemäßen Detektoren geeignet, die sich darauf in hinreichender Menge auf dem Trägermaterial anorden lassen, so daß sie eine hinreichende Speicherfunktion für die zu detektierende Komponente haben. Das können chemisch einwirkende Stoffe sein, z.B. Bariumverbindungen, die mit den zu detektierenden Sulfationen Bariumsulfat bilden, das auf dem Detektor abgelagert wird; es können aber auch physikalisch einwirkende Stoffe sein, wie z. B. Aktivkohle, die mit den zu detektierenden polycyclischen Aromaten sorptive Bindung eingeht. Dazu ist es aber keinesfalls erforderlich, daß die zu detektierenden Stoffe quantitativ auf den Detektoren gesammelt werden müßten. Da es für den jeweils zu detektierenden Stoff zwischen der gebundenen Phase am Detektor und der zu messenden Phase in den meisten Fällen ein Verteilungsgleichgewicht gibt, kann die Konzentration der zu messenden Phase auch in diesen Fallen einfach ermittelt werden. Danach kann je nach Aufgabenstellung auch unbeschichtetes, nichtimprägniertes oder auch völlig unbehandeltes Material, vorzugsweise Fasermaterial, als Detektor für diese Aufgaben eingesetzt werden. Bevorzugtes Einsatzmaterial, das sich für die überwiegende Reihe der erfindungsgemäßen Anwendungen eignet, ist die Zellulosefaser, unabhängig davon, ob sie z. B. aus Holz, Bambus, Baumwolle, Flachs, Lein, Nessel oder dem Papyrus stammt. Das erfindungsgemäße Verfahren ist jedoch darauf nicht beschränkt. Für eine ganze Reihe von Anwendungen werden andere Fasermaterialien als Detektor benötigt, z.B. aus den Materialien Polyolefin, Nylon, Nomex, Teflon, plasmabehandelten Kunststoffen, Keramik oder Metallen, wie Titan, Zirkon, Eisen, Mangan und diese Metalle enthaltenden Legierungen.

Die Fixierung der Wirkstoffkomponenten auf den Trägerstoffen, vorzugsweise faserhaltigen Rohstoffen wie Papier, Zellstoff, Textilien, Fasern, Nonwovens, weniger bevorzugt Partikel kann durch die an und für sich bekannten Prozesse der Feststoffbildung durch Eindampfen zur Trockenen, Ausfällen von Wirkstoffprecursoren oder Fixieren mit einem Adhäsiv geschehen. Sie kann in die Herstellung von faserhaltigen Produkten, z.B. in die Papierherstellung, in die Herstellung von Geweben, Nonwovens, Vliesen oder auch in die Zellstoffherstellung integriert sein oder auch an den fertigen Produkten vollzogen werden. Es ist auch möglich, die Wirkstoffbestandteile im Detektionsprozeß auf dem Detektor zu fixieren.

Typische Beispiele für Wirkstoffprecursor-Paare, die jedes für sich auf Träger applizierbar sind und dann in der wäßrigen Phase miteinander zur Reaktion gebracht werden können, ergeben ebenfalls Wirkstoffbeschichtungen auf den Fasern. Einige Beispiele für derartige Wirkstoffprecursor-Paare, auf die das erfindungsgemäße Verfahren aber nicht beschränkt sein soll, und die daraus generierten Wirkstoffe sind:

| **Wirkstoffprecursor** | **Wirkstoffprecursor** | **Wirkstoff** |
|---|---|---|
| Natriumaluminat | Aluminiumtrichlorid | Aluminiumhydroxid |
| Kaliumpermanganat | Mangan-II-chlorid | Mangandioxid |
| Kaliumpermanganat | Ascorbinsäure | Mangandioxid |
| Gold-III-chlorid | Ascorbinsäure | Goldkolloid |
| Zinksulfat | Calciumsulfid | Zinksulfid |
| Natriumhuminat | Eisen-III-sulfat | Eisenhuminat |
| Natriumsilikat | Aluminiumchlorid | Aluminiumhydroxid |
| Eisen-III-sulfat | Natriumsilikat | Eisen-III-hydroxid |
| Salzsäure | Natriumhuminat | Huminsäure |
| Natriumalginat | Calciumchlorid | Calciumalginat. |

Als Medium, in dem die Umsetzungen mit Wirkstoffprecursor-Paar-beladenen Faserstoffen und gegebenenfalls Partikeln ablaufen, eignet sich auch die Gasphase als Transport-Medium, wenn die Reagenzien, mit und/oder aus denen sich die Wirkstoffe ausfällen lassen, in die gasförmige Phase überführbar sind, z.B. flüchtige Halogenide und flüchtige Basen.

Hilfsmittel bei der Probenahme dienen z.B. dazu, das reduzierende oder oxidierende Milieu im beprobten Medium aufrechtzuerhalten oder unerwünschte Reaktionen an dem Detektor zu vermeiden oder die Detektoren nach erfolgter Probenahme zu stabilisieren. Sie können auch dazu dienen, den Detektor in einen aktivierten Zustand zu versetzen, damit der zu beprobende Stoff überhaupt umgesetzt werden kann.

Ein Beispiel hierfür ist zur Untersuchung des Schwefelgehaltes von trockenen oder nassen Stäuben auf schwefelhaltige Emissionen die UV-Bestrahlung eines Passivsammlers, der ein photolytisch aktivierbares Metalloxid enthält zur Aufnahme von Schwefel enthaltenden Gasen durch Oxidation zu Schwefelsäure, die auf dem Sammler deponiert wird.

Die oxidierende Behandlung als Probenahmehilfsmittel oder zur Probenaufbereitung kann mit flüssigen oder gasförmigen Oxidationsmitteln oder festen löslichen Oxidationsmitteln oder Oxidationsmittel abgebenden Stoffen oder flüssigen. Oxidationsmitteln oder Oxidationsmittellösungen durchgeführt werden. Die reduzierende Behandlung als Probenahmehilfsmittel wird ebenfalls mit gasförmigen oder festen, flüssigen Reduktionsmitteln oder Reduktionsmittellösungen durchgeführt.

Bevorzugte Oxidationsmittel sind Luft, Sauerstoff, Magnesiumperoxid, Ozon, aktivierter Sauerstoff, kaltes Sauerstoffplasma, aktivierter Ozon, atomarer Sauerstoff, Hydroxylradikale, photolytisch generierte Oxidantien, Wasserstoffperoxid, Permanganat, Persulfat.

Die reduzierende Behandlung als Probenahmehilfsmittel oder zur Probenaufbereitung kann mit festen, flüssigen oder gasförmigen Reduktionsmitteln oder festen löslichen Reduktionsmitteln oder Reduktionsmittel abgebenden Stoffen oder flüssigen Reduktionsmitteln oder Reduktionsmittellosungen durchgeführt werden. Bevorzugte Reduktionsmittel sind molekularer Wasserstoff, aktivierter Wasserstoff, atomarer Wasserstoff, Ammoniak, aktivierter Ammoniak, Ascorbinsäure, Hydrazin, Hydrazinhydrat, salz- und legierungsartige Hydride, Unedelmetalle, Chalkogenwasserstoffe, photolytisch generierte Reduktantien und andererseits Schwefelwasserstoff, seine gelösten Salze und gegebenenfalls Schwefelverbindungen, aus denen sich durch Hydrolyse Schwefelwasserstoff und/oder seine gelösten Salze generieren lassen.

Neben den genannten Oxidations-und Reduktionsmitteln können sich je nach Wirkstoffzusammensetzung der Detektoren auch Aktivierungsmittel wie z.B. Säuren oder Laugen, Anlegen einer elektrischen Spannung, Licht, ultraviolettes Licht und ionisierende Strahlen eignen, um Reaktivstoffe in den Detektoren zu generieren bzw. zu aktivieren.

In bestimmten Fallen kann es vorteilhaft sein, die Detektoren mit Reaktionsmitteln, Schutzstoffen gegen Mikroorganismen-Besiedlung, elektromagnetischer Strahlung oder elektrischen Feldern zu beaufschlagen. Hierfür werden Dotierungen der Detektoren mit Silber und/oder Jod vorgezogen.

Die Aktivierungsenergie zur Beschleunigung und/oder Initiierung der gewünschten Generierung der Reaktivstoffe oder die Energie zur Reinigung der Reaktivstoff-Oberflächen der Detektoren oder zu ihrer Desinfektion zur Vermeidung von Bioscaling und Biofouling kann durch Wärme oder Strahlung bis hin zur ionisierenden Strahlung eingetragen werden. Sie kann aber auch ausschließlich durch chemische Reaktion erreicht werden. Tabelle 2 gibt einige Anwendungsbeispiele wieder.

Gase können als Schutzgase zur Vermeidung von Reduktion bzw. Oxidation der Wirkstoffe zugegeben, aber auch als Wirkstoff zur Detektion eingesetzt werden. Dies ist auch bei der Wasseruntersuchung ohne Weiteres möglich, wenn es sich um gasförmige Mittel handelt. Beispiele hierfür sind die Begasung mit Stickstoff, Kohlendioxid, Sauerstoff, Ozon, kalten Plasmen, Chlor und Wasserstoff.

Die Gase können als Druckgase eingesetzt oder durch Elektrolyse erzeugt und als Blasenschwarm angewendet werden. Es ist auch möglich, die Gase durch permeable Schläuche, z. B. Silikonschläuche, in das Wasser einzupressen. Hierbei können die permeablen Schläuche neben den Detektoren angeordnet sein, ohne die Umströmung der Detektoren zu behindern.

Bei geeigneten Untersuchungskompartimenten, die Inhaltsstoffe enthalten, welche die Detektions-Reaktion stören können, zum Beispiel durch reduzierendes Potential, das etwa den Wirkstoff Eisen-III-hydroxid auflösen würde, kann der Detektor zunächst wirkstofffrei sein. Durch gezielte Zugabe oder bereits in dem Kompartiment vorhandenes gelöstes Eisen kann durch Oxidationsmittel das zuvor gelöste Eisen gezielt als Eisen-III-hydroxid-Wirkstoff auf dem Detektor ausgefällt werden und dort seine Funktion als Wirkstoff wahrnehmen. Der Detektor ist somit zumindest mit einem Depot für das Wirkstoffinventar oder Anteile davon und/oder für einen Anteil des Wirkstoffaktivierungsmittelinventars oder Anteile davon derart zur Detektion verbunden, daß das Wirkstoffinventar und/oder das Wirkstoffaktivierungsmittelinventar erst beim bestimmungsgemäßen Gebrauch des Fasermaterials auf die Detektorbestandteile gelangen. Somit kann der Detektor bzw. das Detektionsverfahren dadurch gekennzeichnet sein, daß der Detektor primär wirkstofffrei ist und daß mindestens ein Wirkstoff des Wirkstoffinventars erst in dem zu detektierenden Kompartiment generiert wird. Gasphasen lassen sich für diesen Zweck vorteilhaft mittels semipermeabler Schläuche oder mittels Gasblasen auf den Detektor bringen.

Ähnlich kann bei der Bestrahlung der Detektoren vorgegangen werden, die z.B. in flüssigen Fluiden durch Niederdruck-Ultraviolett-Tauchstrahler erfolgen kann. Auch hier werden die Strahler vorzugsweise so angeordnet, daß sie die Umströmung der Detektoren nicht behindern.

Die Anwendung von elektrischen Feldern zur Anregung katalytischer oder chemischer Prozesse im Festbettfilter ist ebenfalls möglich; dabei werden Verfahren bevorzugt, bei denen die Detektoren selbst oder ihr Inhalt als Elektroden geschaltet sind.

Die geeigneten Wirkstoffe zur Fixierung der zu detektierenden Stoffe in den Detektoren lassen sich ohne Weiteres in der bekannten Weise mit den zur Analyse eingesetzten Fasern, faserhaltigen Materialien, vorzugsweise Papieren oder Fäden nach einer oder mehrerer der obengenannten Methode applizierten. Es kann vorteilhaft sein, die zu detektierenden Substanzen, die in den Detektoren die Kollektion der zu detektierenden Stoffe herbeiführen sollen durch chemische Umwandlung in eine für die Fixierung an den Trägermaterialien geeignete Form zu überführen. Hierzu eignen sich Mineralisierungsreaktionen, die vorteilhaft mit Hilfe von auf den Trägermaterialien fixierten Reaktionsmitteln vorgenommen werden. Hier eignen sich z.B. photoaktivierbare Schwermetalloxide, vorzugsweise diejenigen von Titan, Zirkonium, Mangan, Zink und Eisen und deren Mischoxide, die in der Gegenwart von Oxidationsmitteln und Licht als Hilfsstoffe bzw. Hilfsmittel OH-Radikale generieren können, welche die Zielsubstanz durch oxidierende Mineralisation auf dem Detektor freisetzen, so daß sie dort fixiert werden kann. Es sind aber auch eine Reihe von Enzymen bekannt, mittels derer derartige Mineralisations-Reaktionen auf den Trägern vorgenommen werden können. Beispiele für Verbindungsklassen, die derart auf den Trägern in die gewünschte Form überführt werden können, sind: Phosphorwasserstoff, Phosphorsäureester, halogenorganische Verbindungen, schwefelorganische Verbindungen, Schwefelwasserstoff, arsenorganische Verbindungen, Arsenwasserstoff, metallorganische Verbindungen, organisch komplexierte Metalle und Metalloide, silizium- und bororganische Verbindungen, Schwermetallseifen. Die an und für sich bekannten oxidierenden Mineralisationsreaktionen lassen sich ohne Weiteres sowohl in der wäßrigen als auch in der gasförmigen Phase vornehmen.

In Tabelle 2 werden Detektionsbeispiele mit Hilfsfunktionen gegeben.

Für die Probenahme aus stehenden Gewässern haben sich insbesondere solche erfindungsgemäße Detektoren bewährt, die aktiv mit dem zu beprobenden Medium angeströmt bzw. durchströmt werden.

Aktiv von dem wäßrigen Fluid durchströmte Detektoren können auch innerhalb der zu untersuchenden Flüssigkeit als Festbetten vorgelegt werden, die dann vorzugsweise mit dem zu untersuchenden Fluid angeströmt und/oder durchströmt werden (nicht Teil der beanspruchten Erfindung). Dies kann vor allem durch blasenstrominduzierte Bewegung des Fluids geschehen oder durch eine andere der an und für sich bekannten Methoden der Strömungsgeneration. Die Strömung durch Festbetten kann gerichtet oder ungerichtet sein. Künstlich generierte Strömung kann auch eingesetzt werden, wenn es sich um in der zu untersuchenden Flüssigkeit suspendierte Detektoren handelt, z.B. Glimmerschuppen, Fasern, Glasflakes, Folienwollehäcksel. Durch die Strömung durch die Detektoren hindurch oder an den Detektoren vorbei wird der Detektionsprozeß wesentlich beschleunigt.

Die Relativ-Bewegung der als stückig bis abschnittartig ausgebildeten Fasergebilde oder der als Einzelfasern vorgelegten Fasermaterialien in Bezug auf die zu untersuchende Flüssigkeit kann durch eine oder mehrere der Maßnahmen: Rühren, Pumpen, Bewegen der Einhausung, Durchströmen des Anschwemmfilters, Ausnutzung des Staudrucks bei Nutzung der vorhandenen Wasserströmung, feldaktivierte Bewegung der Fasermaterialien und/oder der Einzelfasern und/oder gegebenenfalls der Partikel herbeigeführt werden.

Es ist aber auch möglich, einen Streifen als Detektor von einer Rolle abzuspulen und kontinuierlich durch die zu detektierende Flüssigkeit hindurchzuziehen, um auf diese Weise eine Relativbewegung zwischen dem Detektor und der zu detektierenden Flüssigkeit herbeizuführen.

Wegen der durch die jeweilige Detektorbewegung relativ zum detektierten Fluid verursachten Änderung des vom Detektor erfaßten Fluidvolumens und der gleichzeitig durch die Bewegung ausgelösten komplexen Änderungen des Detektorverhaltens müssen für den jeweiligen Bewegungsmodus eigene Korrelationsbeziehungen zwischen der Stoffzustands-bzw. Stoffmassen-Änderung am Detektor, der Detektionsdauer und der Stoffkonzentration in dem detektierten Fluid und gegebenenfalls der Eigenbewegung des detektierten Fluids relativ zu der des Detektors bestimmt werden.

Um komplizierte Maßnahmen und Messungen zur Korrelation zwischen Detektionsdauer und durchgesetztem Fluid zu vermeiden, ist es bei einfachen Sorptions- bzw. Reaktionssystemen vorteilhaft, den Detektor so lange mit dem zu vermessenden Kompartiment zu beaufschlagen, bis keine Aufnahme der zu detektierenden Zielsubstanz mehr stattfindet. Diese Art der Detektion ist vorteilhaft bei Systemen, bei denen Gleichgewichtsbeladung erreicht wird. Dies ist in der Regel der Fall, wenn die Detektoren Ionenaustauscher, Schwermetalloxide, Ölfilme auf Trägern oder Aktivkohle enthalten und die Detektion sich z.B. auf die Zielstoffe, Schwermetalle, Aluminium, Fluoride bezieht. Da in der Regel jedoch immer eine konkurrierende Sorption wegen der meistens vorhandenen Vielzahl der ionischen und/oder unpolaren Stoffe, muß von Fall zu Fall getestet werden, welches die beste Vorgehensweise ist.

Die beschriebene Detektionsmethode hat über das bisher Gesagte hinausgehend vorzugsweise zum Ziel, auf dem Detektor alle jeweils relevanten Stoff- und Stoffzustandsparameter gleichzeitig und lückenlos abzubilden.

Bevorzugt ist eine Anordnung mehrerer Wirkstoffe im Detektor in getrennten Positionen, um die gegenseitige Überdeckung von Wirkstoffen zu vermeiden, die zu einem Aktivitätsverlust des Detektors führen könnte. Dazu kann bevorzugt z.B. so vorgegangen werden, daß mit jeweils unterschiedlichen Wirkstoffen beladene Materialien aus länglichen faserhaltigen Gebilden wie Fäden, Papierstreifen, Textilstreifen, Rundgestricke, Drähte einander benachbart angeordnet oder daß in Gewebe eingewebte Fäden mit unterschiedlichen Wirkstoffen eingesetzt werden, die sich zur Analyse leicht von den anderen Wirkstoffen enthaltenden Faden abtrennen lassen.

Der Wirkstoffinhalt des Detektors kann aus einem oder mehreren Wirkstoffen zur Detektion in definiert inhomogener Verteilung in dem Trägermaterial angeordnet sein, und zwar derart, daß der Wirkstoffinhalt in einem vorbestimmten Anteil der das Trägermaterial bildenden Bestandteile in einer vorbestimmten Menge vorliegt und daß der Wirkstoff in einem weiteren vorbestimmten Anteil der das Trägermaterial bildenden Bestandteile nicht oder in einer vom ersten vorbestimmten Anteil der das Trägermaterial bildenden Bestandteile abweichenden Menge und/oder Zusammensetzung vorhanden ist.

Die Anwendung von unterschiedlichen Fasern zur Darstellung multifunktioneller Detektion wird gegenüber der Verwendung unterschiedlicher Partikel vorgezogen, weil die Fasern leicht im Verbundwerkstoff eingesetzt werden können, die Partikel jedoch nicht. Zur Darstellung multifunktionaler Detektionseigenschaften ist es auch möglich, in die Einhausung eines Detektors unterschiedliche Filtermedien mit unterschiedlichen Aufgaben einzufüllen oder auch einen suspendierten Detektor mit unterschiedlichen Filtermedien auszustatten.

Die nachfolgenden Beispiele beschreiben die Erfindung, ohne sie zu begrenzen.

Mittels der im Labor festgestellten Eigenschaften der Detektoren ergeben sich zahlreiche Einsatzmöglichkeiten. Anhand einiger der daraus entwickelten und hier dargestellten Anwendungsbeispiele wird das hohe Anwendungspotential der Detektions-, Kollektions-und Degradations-Varianten des erfindungsgemäßen Verfahrens gezeigt.

Ein besonderes Problem in Industriegesellschaften und bevölkerungsreichen Ländern ist die Überwachung industrieller und kommunaler Abwassereinleiter, um die Vorfluter nicht mit schädlichen Stoffen zu überlasten. Die Einleitung von Abwässern ist in Deutschland durch Regularien wie die sog. Direkt- und Indirekteinleiter-Verordnung geregelt. Dort werden für eine ganze Reihe von Stoffen Höchstwerte genannt. Die kontinuierliche Überwachung der Abwasser-Einleitungen ist mit den herkömmlichen Techniken nicht lückenlos möglich. Im Rahmen der genannten Verordnungen wird die Eigen-Überwachung von den Einleitern durch komplizierte Probenahmetechniken gefordert. Der technische Aufwand, das für die ständige Unterhaltung und Überwachung notwendige Personal, technische Wartung und Reparaturen sowie die hohen Analysenkosten belasten den Eigen-Übervvacher enorm.

Anstelle der direkten Probenahme des Abwassers gibt es nach dem Stand der Technik eine Möglichkeit, die Abwassersituation zu untersuchen. Das ist die sogenannte Sielhaut-Analyse. Die in den Abwasserrohren auf mikrobielle Besiedlung zurückzuführende Beschichtung, die Sielhaut, hat die Eigenschaft, das aus zurückliegenden Emissionssituationen eingetragene Stoffinventar zu speichern. Die Untersuchung der Sielhaut gibt daher gewisse Hinweise auf frühere Betriebsstörungen. Das sind allerdings lediglich sehr vage qualitative Hinweise, die auch nur für schwer abbaubare Schadstoffgruppen auswertbar sind.

Besonders mit dem erfindungsgemäßen Verfahren zur kontinierlichen Überwachung läßt sich die industrielle Abwasser-Eigenüberwachung erheblich vereinfachen, obwohl sich Überwachungsqualität und Auflösung nahezu beliebig steigern lassen. Das kann in dieser Qualität und mit diesem geringen Aufwand mit keinem der bekannten Analysenverfahren dargestellt werden.

Die bevorzugte Variante des erfindungsgemäßen Verfahrens zur Eigenüberwachung ist die Anwendung multifunktionaler faserhaltiger Träger. Bevorzugter Träger ist Papier. Die Eigenüberwachung wird dabei mit mehreren in ihrer Funktionalität unterschiedlichen Papieren gleichzeitig vorgenommen. Das nachstehende Beispiel 1 zeigt eine solche Verfahrensvariante am Beispiel der Überwachung eines Mischabwassers aus der Teerraffinerie und der Epoxidharz-Produktion.

### Beispiel 1: Analytische Überwachung des Mischabwassers aus der Teerraffinerie sowie Epoxid- und Phenolharz-Produktion mit wirkstoffbelegten Papieren als Detektor

Steinkohlen-Teerraffinerie-Abwässer enthalten Phenole, stickstoffhaltige aromatische Basen sowie mono-und polycyclische Kohlenwasserstoffe. Aus der Kokserzeugung enthält das ins Abwasser ablaufende Absitzwasser des Rohteers auch Quecksilber und die Verbindungen flüchtiger Schwermetalle, sowie Ammoniumsulfid, Rhodanid, Cyanid und aromatische Säuren. Ähnliches gilt für die bei der Destillation des Teers anfallende wäßrige Aceotrop- und Reaktionswasserphase. Abwässer aus der Harz-Produktion enthalten Halogenkohlenwasserstoffe, Phenolalkohole und Phenole.

Zur Überwachung wurden nach dem erfindungsgemäßen Verfahren sechs Papiervarianten als Detektor gleichzeitig eingesetzt:
- Kohlenwasserstoffe und Halogenkohlen- Papier mit Aktivkohle wasserstoffe:

- leichtflüchtige Kohlenwasserstoffe und Papier mit Aktivkohle Halogenkohlenwasserstoffe
- Phenole und Phenolalkohole: Papier mit Huminsäuren und Aktivkohle
- stickstoffhaltige aromatische Basen, Papier mit Huminsäuren Ammonium
- Quecksilber und Schwermetalle, Rhodanid, Papier mit Goldfasern Cyanid, Sulfid, aromatische Säuren: sowie Eisen- und Mangan-Hydroxiden
- schwere Teeröle Papier aus Polyethylen-Mikrofasern.

Die Papiere befanden sich auf Vorratsspulen von etwa 10 mm Breite, die nebeneinander auf einer Achse abgespult werden konnten. Die Rollen wurden durch Drehung der Aufwickelspulen verursacht, die ebenfalls für alle Papiersorten auf zwei Achsen angeordnet waren. Die Achsen, auf denen sich die Aufwickelspulen befanden, wurden durch ein Uhrwerk angetrieben. Über Umlenkwalzen gelangten die von fünf Vorratsrollen abgespulten Papierstreifen in ein von dem Abwasser kontinuierlich durchströmtes Meßgefäß. Danach gelangten diese fünf Papierstreifen durch einen Schlitz in das Mikrowellenfeld eines stickstoffdurchströmten Mikrowellenofens, in dem die Papierstreifen bei einer Temperatur von 60 °C innerhalb von 5 min Aufenthaltszeit komplett getrocknet wurden. Das gekühlte Abgas wurde sodann an dem sechsten Meßstreifen vorbeigeführt, der daraus die leichtflüchtigen Komponenten einsammelte und der von der sechsten Vorratsspule abgespult wurde.

Vor dem Aufspulen der getrockneten Papiere auf die Aufwickelspulen wurde dazu jeweils eine viskosestämmige Trennfolie mit aufgespult, um Kontamination durch die exakte Trennung der Wickellagen zu vermeiden. Die Trennfolie erhielt alle 5 cm einen Aufdruck mit Uhrzeit und Datum. Außerdem lief jeder Papierstreifen mitsamt der Trennfolie über eine Schneidewalze, die den Papierstreifen in der Mitte durchtrennte. Die nunmehr getrennten Papierstreifen wurden danach, und zwar jeder für sich, auf Aufwickelspulen gesammelt. Die Aufwickelspulen liefen in einen mit Trockenmittelvorrat versehenen und auf 4 °C gekühlten geschlossenen Behälter, der nur für den Einlauf der Papierstreifen angepaßte Öffnungen besaß.

Für jeden einzelnen der oben genannten Inhaltsstoffe wurden unter Einsatz des Originalabwassers für jedes der verwendeten Papiere Kalibrierkurven mit jeweils dafür geeigneten Meßparametern und Analysenverfahren erstellt, womit die exakte Konzentration der jeweiligen Meßsubstanz auf dem jeweiligen Papier der Konzentration des je zugehörigen Meßparameters im Abwasser zugeordnet werden konnte. Die auf den Aufwickelspulen aufgespulten Detektoren wurden in inertgasgefüllten und entfetteten, verschlossenen Blechdosen als Rückstellmuster im Gefrierschrank aufbewahrt; das jeweilige Gegenstück ging zur Analyse.

Mit dem erfindungsgemäßen Verfahren war es möglich, die Detektoren sowie gegebenenfalls Kapillaren und auch Schläuche eine definierte Zeit in der Meßstelle zu belassen. Durch physiko- oder chemikosorptive Vorgange wurden die Zielsubstanzen an die Wirkstoffe der Detektoren sorbiert. Nach einer gemäß der möglichen Stoffaufnahme kalkulierten Zeit konnte der Detektor aus der Meßzelle entnommen, getrocknet und im Labor chemisch analytisch untersucht werden. Einerseits war eine tiefenabhängige Analyse möglich; inhomogene Belastungen des Grundwassers wurden erkannt. Andererseits wurde über die gesamte Aufenthaltszeit des Detektors in der Meßstelle die Zielsubstanz sorbiert, so daß eine Aussage über die Schadstofffracht während des gesamten Untersuchungszeitraums getroffen werden konnte. Außerdem wurden durch die Anreicherung der Zielsubstanzen auf dem Detektor Aufkonzentrationsfaktoren erzielt, die mit den üblichen Wasseraufbereitungsverfahren (Flüssig/Flüssig-Extraktion, Festphasen-Extraktion) unerreichbar sind: Extraktionen im Labor werden im allgemeinen mit 1 t Wasser durchgeführt, während der Aufenthaltszeit des wirkstoffhaltigen Fasermaterials können jedoch einige 1000 ℓ Wasser das Fasermaterial umströmt haben.

Ein weiterer Verteil ist, daß statt voluminöse Wasserproben lediglich das aufgerollte getrocknete Detektormaterial in das Labor überführt werden mußte. Die Festphasen-Extraktion wurde während der gesamten Aufenthaltszeit des Detektors in der Meßstelle selbsttätig durchgeführt. Es handelte sich quasi um eine "trockene" Grundwasser-Untersuchung. Nach Extraktion der Detektoren konnten die Zielsubstanzen analytisch bestimmt werden. Nach anderen Analyseverfahren können die Detektoren direkt ausgewertet werden oder sie werden zur Analyse verascht. Prinzipiell sind für die Auswertung der Detektoren alle geeigneten Detektionsverfahren eingesetzbar.

### Beispiel 2: Herstellung eines Eisen-III-hydroxidhaltigen Papiers als Detektor mit Polyolefin-Mikrofasern

2,5 g ungebleichter Zellstoff aus Nadelholz wurde mittels Dissolver mit 1,5 ℓ Wasser 25 min lang aufgeschlagen. Dazu wurde eine Lösung von 0,5 g Natriumalginat und 0,5 g Carboxymethylcellulose in 2 t Wasser zugegeben und anschließend unter Rühren mit Calciumchlorid versetzt, bis Koagulation eintrat.

2,5 g gebleichter Zellstoff in der Form eines Blattes wurde mit einer 30-prozentigen Eisen-III-chloridlösung getränkt und antrocknen gelassen. Anschließend wurde er von Hand in Stücke von etwa 3 cm x 3 cm zerrissen und mittels Dissolver in einer verdünnten Sodalösung aufgeschlagen und die neutrale Lösung auf 2 ℓ verdünnt.

5 g LD-Polyethylen-Mikrofasern aus dem Blasspinnverfahren mit Faserdicken unter 3 µm wurde ebenfalls mit Wasser aufgeschlagen und auf 2 ℓ verdünnt.

Die drei erhaltenen Faserdispersionen wurden im Pulper vermischt, danach langsam weitergerührt und auf das Sieb (Blattbildner) zur Papierherstellung aufgegeben. In bekannter Weise wurde daraus Papier hergestellt und mittels 10 cm breiter Doppelwalzen-Schneidvorrichtung zu Filterpapierwolle zerschnitten, die aus 1 mm breiten Filterpapier-Streifen bestand. Die nunmehr erhaltenen Filterpapierwollstränge wurden mit der Papierschere auf Stränge von 25 cm Länge zugeschnitten. Daraus wurden 10 Detektoren (Passivsammler) zu je 2 g von jeweils 25 cm Länge hergestellt, indem die Papierwolle in Polyethylen-Netzschläuche von jeweils 1 cm Durchmesser und 1,5 g Gewicht eingeschweißt wurde. Anschließend wurden die Passivsammler in etwa gleichem Abstand von einer Geländeoberkante in einen Grundwassermeßpegel eingehängt, enthaltend 800 µg/ℓ Blei, 600 µg/ℓ Arsen und 50 µg/ℓ polycyclische Aromaten (gemessen wurden lediglich Naphthalin, Pyren, Fluoranthen und Benzo(a)pyren). Das Grundwasser wies eine Bewegung von 0,25 m/d auf. Die Passivsammler wurden nach einer Detektionszeit von jeweils 1, 2 und 4 Tagen entnommen. Innerhalb des Zeitraums blieb die Proportionalität der aufgenommenen Kontaminanten Arsen, Blei und PAK zur jeweiligen Detektionsdauer konstant. Ebenfalls konstant blieb das Verhältnis Naphthalin zu den übrigen gemessenen Aromaten. Das bedeutet, daß unter den Versuchskonditionen die Naphthalin-Desorption durch Verdrängung aus seiner Sorptionsposition noch nicht stattgefunden hatte.

Ein besonderes analytisches Problem, das bis heute weitgehend ungelöst ist, ist die analytische Erfassung der chemischen Milieuparameter des Kapillarsaumes im Boden oberhalb des Grundwasserspiegels. Mit dem erfindungsgemäßen Detektionssystem in Verbindung mit herkömmlicher einfacher Technik ist es erstmals möglich, einen Großteil dieser Parameter in umfassender Auflösung zu erfassen, wie am Beispiel 3 deutlich gemacht wird.

### Beispiel 3: Untersuchung des Grundwasser-Kapillarsaums im Bereich einer ehemaligen Deponie

Dazu wurde ein 1,5 m langes Edelstahlrohr mit 20 mm Innendurchmesser und 30 mm Außendurchmesser, das mit einer aufgeschraubten Spitze versehen und mit zahlreichen Öffnungen perforiert war, die in regelmäßigen Abständen voneinander die gesamte Rohrwand durchdrangen, senkrecht in das Erdreich eingeschlagen. Es enthielt einen aufschraubbaren Einsatz, der die Schlagenergie des vorzugsweise verwendeten selbsttätigen Schlagwerkzeugs mit Verbrennungsmotor ("Kobra") auf das Rohr übertrug und es dadurch in die Erde triebt. Der innere Hohlraum des Rohres war ausgefüllt mit einem zweiten dünnwandigen Rohr mit 15 mm Innendurchmesser ohne Perforation. Durch jeweiliges Ab-und Aufschrauben des Schlagaufsatzes und jeweiliges Aufschrauben weiterer Rohrsegmente nach dem jeweiligen Einschlagen und dem jeweiligen Einsetzen der Innenrohre wurde die verschraubte Vorrichtung bis in eine Tiefe von 550 cm unter der Geländeoberkante vorgetrieben. Anschließend wurde eine Vorrichtung in die Verrohrung eingeführt, bestehend aus einer Edelstahlhülse mit darin dicht verschlossenem Bleigewicht. Die Edelstahlhülse enthielt eine abschraubbare Klemmvorrichtung, in die als Detektor 21 Detektionspapierstreifen und 81 Detektorfäden eingeklemmt waren. Die Detektionspapierstreifen und die Fäden enthielten aufgedruckte Skalen, die bis auf 0,25 m-Abschnitte aufgelöst waren. Dabei handelte es sich um folgende Anordnung, mit der die dort vorkommenden Verunreinigungen im Kapillarsaum über dem Grundwasserspiegel, der hier in etwa 3,5 m Tiefe angetroffen wurde, untersucht werden sollten. Die Detektionsstreifen waren mittels aufgeklebtem Faden stabilisiert.

Es handelte sich bei den Verunreinigungen um aus einer Altablagerung mit Sickerwasser eluiertes Schadstoffinventar, enthaltend Kohlenwasserstoffe und Halogenkohlenwasserstoffe, Nitroaromaten, Phenole, stickstoffhaltige aromatische Basen, Quecksilber und Schwermetalle, Quecksilber und Schwermetalle, Cyanid, Teeröl. Um diese zu messen, wurden Detektoren gewählt, mit denen diese Parameter untersucht werden konnten:

| | |
|---|---|
| Kohlenwasserstoffe und organische Halogen-verbindungen, Nitroaromaten: | 14 Detektionspapierstreifen mit Aktivkohle |
| | |
| Phenole, Quecksilber, Schwermetalle: | 7 Detektionspapierstreifen mit Huminsäuren und Aktivkohle |
| | |
| stickstoffhaltige aromatische Basen: | 27 Baumwoll-Detektorfäden mit Huminsäure-Imprägnation |
| | |
| Quecksilber und Schwermetalle, Cyanid, Redoxparameter: | 27 Baumwoll-Detektorfäden mit Goldfasern sowie Eisen- und Mangan-Hydroxid-Imprägnation |
| | |
| Teeröle, Redoxparameter: | 27 unbehandelte Baumwoll-Detektorfäden. |

Mittels Hilfsrohr wurde von einer Trommel die Detektoranordnung in das eingeschlagene Meßrohr bis kurz über den Boden abgesenkt; danach wurden die Schutzverrohrung gezogen, die Detektoren auf die notwendige Länge gekappt und am Schraubverschluß des eingeschlagenen Pegels fixiert. Nach 12 Wochen wurde der Pegel gezogen. Direkt nach dem Ziehen wurden die Detektoren freigespült, in Abschnitte einer Länge von 25 cm zerschnitten, in Glasrohre eingeschoben, gekennzeichnet, darin verschlossen und bei minus 20 °C vor Sauerstoff geschützt aufbewahrt.

Nahe der Meßstelle wurde ein zweiter Pegel gleicher Konstruktion und Anordnung eingeschlagen, der als Detektor gemäß Beispiel 1 sauerstoffbegaste Gaze der Vorrichtung (2) enthielt, um die Redoxparameter zu vervollständigen. Nach 12 Wochen wurde der Pegel gezogen, die Gaze von den Silikonschläuchen getrennt und ebenfalls in Abschnitte der Länge von 25 cm zerschnitten, in Glasrohre eingeschoben, gekennzeichnet, darin verschlossen und bei minus 20 °C sauerstoffgeschützt aufbewahrt.

Mit den derart gewonnenen Proben konnte das gesuchte Parameter-Profil in bisher noch nie erreichter Genauigkeit ermittelt werden. Ein Ergebnis dieser Untersuchungen war, daß die Kontamination auf einen anaeroben Bereich begrenzt ist, der von wenigen cm unterhalb des Grundwasserspiegels bis 1,6 m über dem Grundwasserspiegel reichte.

Das hier mitgeteilte Beispiel (3) der Anwendung des erfindungsgemäßen Verfahrens zur Untersuchung unterirdischer Aquifere macht durch das Detektionsverfahren eine sehr genaue Modellierung der Ausbreitung und Bewegung von Kontaminanten darin möglich und erlaubt eine exakte Berechnung und Plazierung notwendiger Gegenmaßnahmen. Damit kann nicht nur auf der Seite des analytischen Überwachungs- und Ermittlungs-Aufwandes und des Pegelbaus viel Geld eingespart werden, sondern auch an Präventiv- und Sanierungsmaßnahmen wie bei der Anlage von Schlitzwänden, Barrieren, Reaktiven Wänden, Funnel-and-Gate-Systemen, hydraulischen Maßnahmen usw.

Immer wieder kommt es in der Lebensmittelindustrie oder auch Tierfutterindustrie zu Skandalen, weil z. B. dioxinhaltige industriestämmige Altöle in Tiernahrungsmittel geraten oder Fungizide in Rezeptur-Bestandteile, z.B. von Coca-Cola. Durch kontinuierliche Analyse von Produkten und Rezeptur-Komponenten könnten solche Probleme vermieden werden. Mit der am Markt vorhandenen Technik sind solche Detektionstechniken zwar möglich, aber sehr kostenaufwendig. Durch das erfindungsgemäße Verfahren werden Analysentechniken verfügbar, deren Anwendung sehr einfach ist und deren Überwachung lückenlos durchgeführt werden kann. Mit kontinuierlicher Hindurchführung eines erfindungsgemäßen Detektionspapiers oder Detektionsfadens durch das natürliche wäßrige Medium, durch Abwässer, Produkte, Rohstoffe und Zwischenprodukte kann man a) lückenlose Rückstellmuster generieren und b) lückenloses Detektor-Material für die Qualitätskontrolle gewinnen. Beispiele hierfür sind die Wasser- und Abwasseruntersuchung, die Weinerzeugung, Pommes-frites-Öl sowie die Bier- und Getränke-Produktion.

Das erfindungsgemäße Detektionsverfahren eignet sich sehr gut zur Untersuchung der natürlichen und künstlichen Aquifere Grundwasser, Kanäle, Stauseen, Fließgewässer, Binnenseen, Abwasserkanäle und Abwasserbehandlungsanlagen, Häfen, Meere und der an die Gewässer angrenzenden Kompartimente. Für Binnengewässer besteht der Detektor vorzugsweise aus einem oder mehreren streifen- und/oder fadenförmigen Bestandteilen, denen eine makroskopische Reiativbewegung zu dem fluiden und/oder partikulären Meßmedium aufgeprägt ist, die aber zueinander keine makroskopisch erkennbare Relativbewegung ausführen.
Die Detektoren enthaltend Fäden und/oder Streifen oder in Körben als Formkörper, gebrochene Partikel oder zu Streifen zerteilte Fasermaterialien können vorteilhaft in natürlichen oder künstlichen Fließgewässern eingesetzt werden. Zum Einsatz der Detektoren eignen sich die verschiedensten Kompartimente, wie z.B. Kapillarsaum, Grundwasser, Kommunal-, Industrie- und Kraftwerks-Abwasser, Kühlwasser, Kreislaufwasser, Grubenwasser, Oberflächengewässer, Fluß, Strom, Binnensee, Schelfmeer, Ozean, Getränke, staubförmige und/oder stückige Lebensmittel, flüssige oder schmelzbare Lebensmittel, flüssige, staubförmige oder stückige Industrie-Produkte, Erdreich, flüssige und staubförmige Rohstoffe und Zwischenprodukte, Altablagerungen, Erdreich, Sediment, Watten, Kompostrotten, Deponien, Altstandorte, Altlager.

**Tabelle 1: Beispiele für Detektoren zur Detektion von Stoffen**

| **detektierter Stoff, daraus erzeugtes/ abgebautes Detektionsmittel** | **Detektor-Wirkstoffe** |
|---|---|
| Quecksilber als Amalgam | Goldfasern, Blattgold, vergoldete oder versilberte Aktivkohle, vergoldeter oder versilberter Ruß, Goldkolloid |
| | |
| Quecksilber als Sulfid | Bariumsulfat mit biologisch verwertbarem Reduktionsmittel |
| | |
| Nach der elektrochemischen Spannungsreihe edlere Metalle als Zink, als Metalle | Zinkstaub, Zinkspäne |
| | |
| Verbindungen von Gold und Silber, als Metalle | Aktivkohle, Huminsäure Erdalkalihuminat |
| | |
| Aluminium, Schwermetalle, Actiniden und Edelmetalle, Radionuklide der Schwermetalle und Erdalkalien als Carboxylate und Edelmetalle | Huminsäure, Erdalkalihuminat Alginsäure, Fett-und Ölsäuren |
| | |
| Schwermetalle, Arsen, Antimon Wismut, Tellur als Sulfide oder Disulfide | Bariumsulfat plus biologisch verwertbares Reduktionsmittel |
| | |
| Schwermetalle als Mercaptide | Mercaptane |
| | |
| Schwermetalle*, Selen-und Tellurverbindungen, Sauerstoff enthaltende Verbindungen von Arsen, Antimon, Wismut, Tellur als Einlagerungsverbindungen oder Elemente * z.B. Cadmium, Quecksilber, Nickel, Kupfer, Cobalt, Eisen | Eisen-III-oxide und-hydroxide; Mangan-III- undMangan-IV-oxide und -hydroxide |
| | |
| Gerbstoffe, N-heterocyclische aromatische Polycyclen, aromatische Amine Phenole, Alkaloide als Eisen-Komplex-Verbindungen, Huminstoffe | Aluminiumhydroxid ; Eisen plus Oxidationsmittel ggf. mit sorptions-u/o korrosions beschleunigenden Zusätzen (z. B. Kupfer, Cadmium, Eisen, Blei; aber auch Aluminium, Gallium, Indium, Thallium, Blei, Silber) |
| | |
| Schwefelverbindungen als Baryt | Bariumcarbonat plus biologisch verwertbare Oxidationsmittel; Barium-carboxylate komplexer organischer Säuren (z.B. Huminate, Alginate, Oleate) plus Oxidationsmittel |
| | |
| Elementare und oxidierte ionische Halogenverbindungen als Silberhalogenid | silberhaltige Aktivkohle ggf. plus Reduktionsmittel |
| | |
| Cobalt, Zink als Dithizonat (Entsprechend können eine Reihe von Kationen mit dithizonorganischen Verbindungen entsprechenden Reaktions mustern schwerlösliche Verbindungen bilden. Fällungsreaktionen sind dann für den erfin dungsgemäßen Zweck geeignet, wenn sich die organischen Reaktanten in weitgehend wasserunlöslicher Form in den faserhaltigen Detektoren binden lassen; wie hier am Bei spiel des Dithizons gezeigt wird.) | Aktivkohle-oder makroporöse Polyolefinpartikel plus Dithizon; oder Paraffinöl-Dithizon-Lösung |
| | |
| Calcium, Strontium, Barium, Radium, Gallium, Indium, Thallium als Carboxylate | Huminsäuren, langkettige Aluminium-Carboxylsäuren, Alginsäuren |
| | |
| Blei als Bleisulfid Quecksilber als Quecksilbersulfid Kupfer als Kupfersulfid Silber als Silbersulfid Nickel als Nickelsulfid | Zinksulfid |
| | |
| Magnesium als Carboxylat Radium als Carboxylat | Huminsäuren, langkettige Carboxylsäuren |
| | |
| Astat, Jod, Jodide mit Oxidationsmittel, Jod als Radionuklid als Sorptions-und Einlagerungsverbindung | Stärke, Polysaccharide, Aktivkohle |
| | |
| Reduktionspotential ergibt bei Reduktion HgS | Quecksilberchlorid plus Baryt |
| | |
| Reduktionspotential ergibt bei Reduktion AgS | Silberchlorid plus Baryt |
| | |
| Reduktionspotential ergibt bei Reduktion PbS | Bleisulfat |
| | |
| Reduktionspotential wird bei der Reduktion gelöst | Baryt |
| | |
| Reduktionspotential ergibt bei Reduktion Fe(OH)₂ x Fe(OH)₃ oder wird ganz aufgelöst als Hydrogen-carbonat oder zu Pyrit umgewandelt | Eisen-III-hydroxid |
| | |
| Reduktionspotential ergibt bei Reduktion Pyrit | Eisen-III-hydroxid plus Baryt |
| | |
| Oxidationspotential ergibt bei Oxidation Fe(OH)₃, | Pyrit, Eisensulfid |
| Oxidationspotential ergibt bei Oxidation PbSO4, Pyrit und/oder Eisensulfid plus Bariumcarbonat | Bleisulfid |
| | |
| Oxidationspotential ergibt bei Oxidation Fe(OH)₃ + BaSO₄ | Bariumcarbonat plus Elementarschwefel |
| | |
| Oxidationspotential ergibt bei Oxidation BaSO₄ | Bleisulfid plus Bariumcarbonat |
| | |
| Oxidationspotential ergibt bei Oxidation Mangan-III- und/oder Mangan-IV-Hydroxide | Mangancarbonat |
| | |
| Oxidationspotential ergibt bei Oxidation Psilomelan | Mangancarbonat plus Bariumcarbonat |
| | |
| Dioxine PAK (polycyclische aromatische Kohlenwasserstoffe), halogenorganische Verbindungen als sorbierte Phase, Radon | Polyethylen-Mikrofasern, Ruß, Aktivkohle, schwermetallhaltige und/oder sulfidhaltige und/oder pyrithaltige Aktivkohle |
| | |
| Chromat als Bariumchromat und Chromsäure | Bariumhuminat Bariumalginat |
| | |
| Fluorid als Calciumfluorid oder an Mischoxide gebunden | Calciumhuminat, Calciumalginat Aluminium,-Eisen-und/oder Mangan-Verbindungen als Mischoxide oder Huminate |
| | |
| Chlorid, Bromid, Jodid, Cyanid als Silberhalogenid bzw. Silbercyanid | Silberhuminat, Silberoxid-Misch kristalle mit Eisen-und/oder Mangan-Sauerstoff-Verbindungen |
| | |
| halogenorganische Verbindungen als dehalogenierte Verbindungen | schwermetallhaltige Aktivkohle |
| | |
| Krypton, Xenon, Radon, Schwefelhexafluorid als Sorbate | Aktivkohle |
| | |
| Sulfide, Schwefelwasserstoff, Mercaptane, Schwefelkohlenstoff, Thiokohlensäuren als Disulfide, Elementarschwefel oder Sulfat | Aktivkohle, alkalisiert, oder jodiert; anorganische und organische Verbindungen der Lanthanide, der Actinide, des Yttriums und Scandiums; schwermetallhaltige Aktivkohle; schwerlösliche Bariumcarboxylate; Oxidationsmittel |
| | |
| Nitroverbindungen als Aminoverbindungen | schwermetallhaltige Aktivkohle; |
| | |
| monomere oder polymere Diazoverbindungen | Eisen, Zink, palladiniertes Eisen; Wasserstoff |
| | |
| Fluorid als Sorptionskomplex, Huminstoffe | Aluminiumhydroxid |
| | |
| partikuläre hydrophile Schwebstoffe als an den Fasern adhärierte Schwebstoffe | Cellulosefasern; Cellulosefasern plus Polysaccharide; Cellulosefasern plus Eisenhydroxid |
| | |
| partikuläre hydrophobe Schwebstoffe als an den Fasern adhärierte Schwebstoffe | Polyolefinfasern, Polyolefinfasern plus Ruß |
| | |
| emulgierte Ölschwebstoffe als an den Fasern adhärierte Schwebstoffe | Polyolefinfasern, Polyolefinfasern plus Ruß |
| | |
| Phenole, aromatische Amine, stickstoffhaltige aromatische Heterocyclen, Nitrile, Alkaloide als chemisch gebundene Addukte | Huminsäuren, Alginsäuren, saure Ionenaustauscher, schwerlösliche Eisensalze und Eisenoxide |
| | |
| Halogen-, Chalkogen-, Phosphor- und Arsen-organische Verbindungen, Metallorganische Verbindungen als Sulfate, Selenate, Selenite, Phosphate, Arsenate, Arsenite, Halogenide, Metall-Sauerstoff verbindungen | kontinuierlich oder diskontinuierliche mit UV-Strahlung bestrahlte Titan-, Eisen- und Mangan-Mischoxide; Oxidationsmittel |
| | |
| Halogen-, Chalkogen-, Phosphor-und Arsen-organische Verbindungen; Metallorganische Verbindungen als Sulfate, Selenate, Selenite, Phosphate, Arsenate, Arsenite, Halogenide, Metall-Sauerstoffverbindungen | die oxidative Mineralisation beschleunigende Enzyme, Oxidationsmittel |

Eine wichtige Domäne für den Einsatz des erfindungsgemäßen Verfahrens mit den erfindungsgemäßen Detektoren sind die Bereiche heutiger und ehemaliger Radionuklid-Anwendung. In Frage kommen z.B. betriebene und stillgelegte Kernkraftwerke, Brennelemente-Wiederaufbereitungsanlagen, radionuklidhaltige Abwässer aus Klein- und Großkliniken sowie Forschungseinrichtungen, Radionuklid-Altlasten und - Altablagerungen sowie betriebene und ehemalige Uran-, Thorium- und Monazit-Abbau- und Aufbereitungs-Lokalitäten (Beispiele: stillgelegte Brutreaktoren, Kernwaffentestgelände, Anreicherungsanlagen, Tschernobyl, Hanford, Fernald, Wismut-Gelände).

Besonders bedeutend ist im Kernkraftwerk der Einsatz der erfindungsgemäßen Detektoren als Analyseninstrument zur kontinuierlichen Überwachung von Wasserparametern. Der wesentliche Bedarf dafür wird im Kernkraftwerk in der kontinuierlichen Überwachung von Betriebswasser-Kreisläufen, Abwasserabläufen, Grundwassermeßstellen und Dampfkreisläufen gesehen. Für diesen Zweck wird der Detektor in Form von "endlosen" Fäden, Papierbändern oder Textilbandern eingesetzt. Aber auch hier ist es möglich, die wirkstoffhaltigen Fasern als solche ungeformt einzusetzen. Die an den geeigneten Sorbentien als Radionuklid-Kollektoren nach vorgewählter Einwirkungszeit gespeicherten Radionuklide lassen sich hervorragend mit den bekannten Methoden detektieren. Dank der entwickelten Methode gelingt es mit einer gemäß dem Stand der Technik nicht erreichbaren Präzision hinsichtlich Örtlichkeit (Meßpegel), zeitlicher Auflösung und Quantität, selbst geringste Spuren von Radionukliden zu detektieren und lückenlos zu dokumentieren. Das hat den Vorteil, etwaige Undichtigkeiten oder sonstige Störungen weit früher zu erkennen, als es mit herkömmlichen Detektionstechniken möglich ist. Das kann den Betreiber aber auch in die Lage versetzen, notwendige Maßnahmen unter geringerem zeitlichem Druck einzuleiten, als das bisher möglich war. Auch hier lassen sich die Eigenschaften der erfindungsgemäßen Sorbentien als Detektionsmaterial nutzen.

Für die Detektion von instabilen langlebigen Elementen (beispielsweise Cobalt 60, Nickel 63, Strontium 90, Barium 133, Radium 226, Titan 44, Aluminium 26, Thallium 204, Blei 210, Antimon 125, Jod 131, Wismut 207, Thorium 232, Promethium 147 und den übrigen Actiniden-und Lanthanidenstrahlern, Krypton 85 und Radon 222) wird vorzugsweise ein Fasermaterial eingesetzt, das 20 % Eisen-III-oxide, 5 % Mangan-III/IV-oxide, 10 % Aktivkohle und 15 % Huminsäure enthält.

Die kontinuierliche Produkt-Überwachung, Prozeßüberwachung und Eduktkontrolle durch möglichst lückenlose Musterrückstellung und Analytik sind wichtige Aspekte des Qualitätsmanagements auch in industriellen Fertigungsprozessen; unabhängig davon, ob sie sich auf wäßrige oder nichtwäßrige Medien beziehen. Auch hier kann das erfindungsgemäße Verfahren zur Musterrückstellung und Analytik mit diesen Detektoren Erleichterung bringen. Es ist ohne Weiteres möglich, die Detektoren mit geeigneten Wirkstoffen zu dotieren bzw. zu imprägnieren, um eine hinreichend empfindliche kontinuierliche Qualitätsüberwachung des betreffenden flüssigen Gutes zu erreichen.

Auch für den Zweck der Boden- und Sedimentuntersuchung lassen sich die erfindungsgemäßen Detektoren vorteilhaft einsetzen. Dazu können die Detektoren in den Boden eingelegt und mit Boden abgedeckt werden. Sie können auch auf den Boden aufgelegt und zum besseren Kontakt z.B. mit Beschwerungsmitteln wie Folien, Sand, Bretter, Kies, Splitt, Formsteine abgedeckt werden. Ähnlich kann man verfahren mit Erdreichmieten, Altablagerungen, Hafenschlamm-Poldern, Sedimenten, Watten, Kompostrotten, Erdreichwäsche-Rückständen, Rüstungs- und Kampfstoff-Altstandorten sowie -Ablagerungen und -Altlägern, sonstigen Altstandort-Geländen und sonstigen Verdachtsflächen mit Fluid-Feststoff-Konglomeraten.

**Tabelle 2 : Beispiele zur Detektion von Stoffen unter Hilfsstoffanwendung**

| **Zu detektierender Stoff** | **Wirkstoff im Detektor** | **Hilfsmittel zur Detektion** |
|---|---|---|
| Sulfid, Hydrogensulfid, Schwefelwasserstoff, Polysulfid, | Eisen-III-oxide u.-hydroxide, Mangan-III-undMangan-IV-oxide und-hydroxide, Kupfer-oxide und-hydroxide, | Luft, Permanganatlösung Wasserstoffperoxid Aktivierte gasförmige UV-Bestrahlung |
| | | |
| Nitrat, | Anionenaustauscher, | Wasser, Laugen, Säuren |
| Sulfat; Phenole, aromatische Amine, Nitroaromaten, Quecksilber, Schwermetalle, Aluminium, Metalloide; | Zinkmetall, Eisenmetall, Silbermetall, Kupfermetall, Goldkolloid | Galvanisierung, Reduktionsmittel, Photolyse |
| | | |
| organisch substituierte Mercaptide/Mercaptane Rhodanide, Cyanide, Cyanate | Eisen-III-oxide und -hydroxide, Mangan-III- und Mangan-IV-oxide und -hydroxide, Kupferoxide und -hydroxide, Zinkoxide und -hydroxide, | Oxidationsmittel Mikrowellen Infrarotstrahlen Wärme UV-Strahlen Ionisierende Strahlen |
| | | |
| Schwefelkohlenstoff, Thiocarbonate, Rhodanide, Cyanide, Cyanate, Di- und Trithiocarbonate | Eisen-III-oxide und -hydroxide, Mangan-III- und Mangan-IV-oxide und -hydroxide, Kupferoxide und -hydroxide, Zinkoxide und -hydroxide in Kombination mit Gruppe-3-Elementen | Oxidationsmittel Mikrowellen Infrarotstrahlen Wärme UV-Strahlen Ionisierende Strahlen |
| | | |
| Nitrat | Palladiumhydrid | Luft |
| | | |
| Nitroorganische und halogenorganische Stoffe | Zinkmetall, Eisenmetall | Photolyse Permanganatlösung Wasserstoff |
| | | |
| Schwermetalle Metalloide | Eisen-III-oxide und -hydroxide, Mangan-III-und Mangan-IV-Eisen-III-oxide und -hydroxide Kationenaustauscher Schwermetallsulfide | Luft, Permanganatlösung, Sulfide, Hydrogensulfide Schwefelwasserstoff Säuren |

Die Detektoren lassen sich ebenso dazu verwenden, zusätzlich zu der Abbildung bestimmter Stoffzustände in der untersuchten Lokalität oder auch völlig unabhängig davon, Richtungs-und Geschwindigkeitsparameter der Fluidströmung zu vermessen. Neben den beschriebenen, nahezu umfassenden Detektionsmöglichkeiten von Stoffen und Stoffzuständen und den Möglichkeiten zur kontinuierlichen und Probensammlung lassen sich mit den Detektoren überraschend auch makroskopische Strömungsvorgänge in Flüssigkeiten feststellen und dokumentieren, die in dieser Auflösung bislang nicht möglich waren. Dies gilt besonders für Grundwasserbewegungen, die bisher nur im groben Raster mittels zahlreicher Meßpegel feststellbar waren.

Die Untersuchung der hydraulischen Bewegungen des zu untersuchenden Fluids mit den erfindungsgemäßen Detektoren kann in der Regel direkt mit der Untersuchung der Stoffzustände und stofflichen Inhalte des Fluids verknüpft werden. Zur Untersuchung der Bewegungsvorgänge werden vorzugsweise hinreichend gespannte Fäden oder schmale Bänder eingesetzt.

Besonders bevorzugte Wirkstoffe in den Detektoren sind Oxide und Mischoxide und Hydroxide von Eisen, Mangan, Titan, Silicium und Aluminium, die Polysacharide und ihre natürlichen und künstlichen Derivate und Salze, die Huminstoffe und ihre Salze, Polyacrylsäuren und ihre Salze, polymere Amine und ihre Salze, Chitosan und seine Salze, organische Flockungsmittel und ihre Salze, alle schwerlöslichen Oxide und Hydroxide, Aktivkohlen und Cellulose sowie ihre chemisch modifizierten Derivate, zum Ionenaustausch fähige Stoffe, zur Komplexbildung mit metallischen und nichtmetallischen Elementen, Molekülen und ionisierbaren Stoffen fähige Stoffe, Enzyme, Aktivkohlen, Edelmetalle, Metallhydride, amalgamierbare Metalle und Metallhydride.

## Patentansprüche

1. Detektor im offenen System zur Ermittlung der Art und/oder des Gehalts von vorbestimmten Substanzen und/oder zur Ermittlung von Richtungs- und/oder von Geschwindigkeits-Parametern an einem Untersuchungsort in Gewässern, wobei das Medium während der Untersuchungsdauer, nämlich einem vorbestimmten Zeitraum von mehr als einer Stunde, passiv ein Volumenelement des im offenen System angeordneten Detektors um- oder durchströmt, wobei dieser ein wenigstens einen Wirkstoff aufweisendes Trägermaterial enthält, wobei der wenigstens eine Wirkstoff auf dem Trägermaterial angeordnet und/oder darin enthalten und im Untersuchungsmedium durch Einwirkung vorbestimmter Substanzen, die am Untersuchungsort vorkommen, auf vorbestimmte Weise veränderbar ist und wobei der wenigstens eine Wirkstoff in einer für die Untersuchungsdauer ausreichenden Menge vorhanden ist, **dadurch gekennzeichnet,**
• **daß** die Packungsdichte des, wirkstoffautweisenden Trägers weniger als 100 g/ℓ beträgt, wobei der Träger als faser- oder wolleförmiges Trägermaterial vorliegt,
• **daß** für die linien-, flächen- oder volumenförmige Untersuchung von großräumigen wäßrigen Systemen die Form der Träger so gewählt ist, daß die eine Raumdimension gegenüber den beiden anderen Raumdimensionen überwiegt oder daß zwei Raumdimensionen gegenüber der dritten Raumdimension überwiegen,
• wobei der wirkstoffaufweisende Träger als Packung in einer Enhausung enthalten ist, die eine der Formen: lang und schlauchförmig, lang und rohrförmig aufweist und wobei die Einhausung auf mindestens einer Seite hydraulisch durchlässig ist
• und **daß** der Träger nur schwerlösliche Wirkstoffe enthält und im Untersuchungsmedium in Form einer Anordnung oder Dispersion von faserförmigen Teilchen vorliegt.

2. Detektor nach Anspruch 1, **dadurch gekennzeichnet, daß** mindestens ein Wirkstoff [zur Detektion] im Detektor in Form einer Beschichtung und/oder Imprägnierung definiert inhomogen derart verteilt ist, daß der Wirkstoff in einem vorbestimmten Anteil der das Fasermaterial bildenden Fasern in einer vorbestimmten Menge vorliegt und daß der Wirkstoff in einem weiteren vorbestimmten Anteil der das Fasermaterial bildenden Fasern nicht oder in einer vom ersten vorbestimmten Anteil der das Fasermaterial bildenden Fasern abweichenden Menge vorhanden ist.

3. Detektor nach Anspruch 1, **dadurch gekennzeichnet, daß** ein oder mehrere der Wirkstoffe [zur Detektion] in definiert homogener Verteilung in einer oder mehreren der Formen Imprägnierung und/oder Beschichtung von Fasern enthalten ist.

4. Detektor nach einem der Ansprüche 1 bis 3, **dadurch gekennzeichnet, daß** die Einhausung aus mindestens einer der Erscheinungsformen: Netz, Folie, Textil, Gewebe, Vlies, Papier, Nonwoven, perforiertes Rohr, Verbundmaterial, Gestrick, Drahtgitter, Lochblech besteht.

5. Detektor nach einem der Ansprüche 1 bis 4, **dadurch gekennzeichnet, daß** er feldaktivierbare Hilfsstoffe enthält, die durch mindestens eine der Feldwirkungen: Gravitationsfeld, Magnetfeld und elektrisches Feld aktivierbar sind.

6. Detektor nach einem der Ansprüche 1 bis 5, **dadurch gekennzeichnet, daß** er eine Vorrichtung enthält, die mindestens eine der Funktionen aus folgender Gruppe aufweist: Zusammenhalten des Detektorinhalts, Schutz des Detektorinhalts gegen Zerfall, Formgebung, Strömungsleitung, Fixierung, Auftriebsvermittlung, Abtriebsvermittlung, elektrische Isolation, Einhausung der Detektorbestandteile.

7. Verfahren im offenen System zur Detektion der Art und/oder des Gehalts von vorbestimmten Substanzen und/oder zur Ermittlung von Richtungs-Parametern an einem Untersuchungsort in Gewässern, wobei das Medium während der Untersuchungsdauer ein Volumenelement eines Detektors nach einem der Ansprüche 1 bis 6 während mehr als einer Stunde passiv um- oder durchströmt
**dadurch gekennzeichnet, daß** der Detektor im Wege der passiven Probenahme in das Untersuchungsmedium eingebracht und darin während der Untersuchungsdauer belassen wird, worauf der Detektor entnommen wird und die Bestimmung der Menge des wenigstens einen Wirkstoffs und/oder der durch Einwirkung der vorbestimmten Substanz und/oder der Strömung des Flüssigkeitsstroms auf den wenigstens einen Wirkstoff entstandenen Reaktionsprodukts als Maß für die Art und gegebenenfalls die Menge der vorbestimmten Substanz und/oder der Strömung des Flüssigkeitsstroms erfolgt.

8. Verfahren nach Anspruch 7, **dadurch gekennzeichnet, daß** ein Detektor aus mehreren streifen-, faden- und/oder bandförmigen Bestandteilen eingesetzt wird, denen eine Relativbewegung zu dem fluiden Meßmedium aufgeprägt ist.

9. Verfahren nach einem der Ansprüche 7 und 8, **dadurch gekennzeichnet, daß** in dem Detektor, zumindest aber in seinem Einflußbereich, zusätzlich zu den herrschenden Strömungsparametern oder anstelle der herrschenden Strömungsparameter innerhalb des Untersuchungs-Kompartiments eine von den herrschenden Strömungsparametern abweichende Flüssigkeitsströmung oder eine Bewegung des Untersuchungsmediums relativ zu dem Detektor generiert wird.

10. Verwendung des Detektors nach einem der Ansprüche 1 bis 6 in einem offenen System zur Ermittlung des Gehalts von vorbestimmten Substanzen und/oder zur Ermittlung von Richtungs- und/oder von Geschwindigkeits-Parametern in Gewässern, wobei das Medium ein natürliches oder künstliches Aquifer, Grundwasser, ein Kanal, ein Stausee, ein Fließgewässer, ein Binnensee, ein Abwasserkanal, eine Abwasserbehandlungsanlage, ein Hafen, oder ein Meer ist.

## Claims

1. Detector in open system to determine the kind and/or contents of predetermined substances and/or to determine directional and/or velocity parameters at an examination site in waters where for the duration of the examination, namely a prefixed period of time of more than one hour, the medium flows passively round or through a volume element of the in the open system arranged detector containing a support of at least one agent, with this at least one agent being arranged on and/or in the support and being changeable in a predetermined way in the medium under examination by the action of predetermined substances occurring at the examination site, and with the at least one agent being available in a quantity sufficient for the duration of the examination, **characterized in that**
- the packing density of the agent-keeping support is less than 100 g/ℓ and the support is of fibre- or wool-shaped material,
- for the linear, areal, or volume examination of large-scale aqueous systems the shape of the supports is chosen such that one spatial dimension prevails over the two other spatial dimensions, or that two spatial dimensions prevail over the third special dimension,
- with the agent-keeping support being contained in a casing of one of the shapes: long and tubular, long and pipe-shaped and with the casing being hydraulically permeable on at least one side,
- and that the support does only contain slightly soluble agents and is present in the medium under examination in form of an arrangement or dispersion of fibre-shaped particles

2. Detector according to Claim 1, **characterized in that** at least one agent [for detection] defined in form of a coating and/or impregnation in the detector is inhomogeneously distributed such that the agent is present in a predetermined quantity in predetermined portion of the fibres forming the fibre material, and that in another predetermined portion of the fibres forming the fibre material the agent is present not at all or in a quantity deviating from the first predetermined portion of the fibres forming the fibre material.

3. Detector according to Claim 1, **characterized in that** one or more of the agents [for detection] are in a defined homogeneous distribution contained in one or more of the forms of impregnation and/or coating of fibres.

4. Detector according to one of the Claims 1 to 3, **characterized in that** the casing consists of at least one of the design forms: net, foil, textile, woven, fleece, paper, nonwoven, perforated tube, composite material, knit fabric, wire grating, perforated plate.

5. Detector according to one of the Claims 1 to 4, **characterised in that** it contains accessory substances to be activated by fields and that can be activated by at least one of the field actions: gravitation field, magnetic field, and electric field.

6. Detector according to one of the Claims 1 to 5, **characterised in that** it contains an appliance showing at least one of the functions out of the following group: keeping together of the detector contents, protection of the detector contents against disintegration, shaping, flow conduction, fixing, buoyancy force, descending force, electric insulation, casing of the detector components.

7. Procedure in an open system to determine the kind and/or contents of predetermined substances and/or to detect directional parameters at an examination site in waters where for the duration of the examination the medium flows during more than one hour passively round or through a volume element of a detector according to one of the Claims 1 to 6, **characterized in that** the detector by way of passive sampling is placed in the medium under examination to remain there for the duration of the examination, after which the detector is removed to then determine the quantity of the at least one agent and/or of the reaction product originated by the action of the predetermined substance and/or the flow of the liquid current upon the at least one agent, so defining the kind and, if so, the quantity of the predetermined substance and/or the flow of the liquid current.

8. Procedure according to Claim 7, **characterized in that** a detector is used that consists of several strip-shaped, filiform and/or ribbon-shaped components on which is impressed a relative motion to the fluid measuring medium.

9. Procedure according to one of the Claims 7 and 8, **characterized in that** within the detector, yet at least within its sphere of influence, in addition to the existing flow parameters or instead of the existing flow parameters within the examination compartment is generated a liquid current deviating from the existing flow parameters, or a movement of the medium under examination relatively to the detector.

10. Use of the detector according to one of the Claims 1 to 6 in an open system to determine the contents of predetermined substances and/or to determine directional and/or velocity parameters in waters, with the medium being a natural or artificial aquifer, ground water, a channel, an impounding reservoir, flowing waters, an inland lake, a sewer duct, a wastewater treatment plant, a harbour, or a sea.

## Revendications

1. Détecteur dans un système ouvert pour déterminer le genre et/ou le contenu des substances prédéterminées et/ou pour déterminer des paramètres de direction et/ou de vitesse à un lieu d'examen dans des eaux où pendant la durée de l'examen, à savoir une période donnée de plus d'une heure, le milieu passe passivement par ou autour d'un élément volumétrique du détecteur placé dans les système ouvert et contenant un porteur avec du moins un agent, avec ce du moins un agent étant placé sur et/ou dans le support et changeable d'une manière prédéterminée dans le milieu examiné par l'action des substances prédéterminées se trouvant au lieu d'examen, et où ce du moins un agent existe d'une quantité suffisante pour la durée d'examen, **caractérisé en ce que**
- la densité de tassement du support porteur-agent est sous 100 g/ℓ et le support est d'un matériel en forme de fibre ou de laine,
- pour l'examen linéaire, d'aire ou de volume des systèmes aqueux de grandes dimensions la forme des supports est choisie telle qu'une dimension spatiale dépasse les deux autres dimensions spatiales, ou que deux dimensions spatiales dépassent la troisième dimension spatiale,
- avec le support porteur-agent étant emboîté dans une caisse d'une des formes : longue et tubulaire, longue et tuyau-forme, et au moins une côté de la caisse étant hydrauliquement perméable,
- et que le porteur ne contient que des substances peu solubles et existe dans le milieu examiné en forme d'un arrangement ou dispersion de particules de forme de fibre.

2. Détecteur selon la Revendication 1, **caractérisé en ce que** dans le détecteur du moins un agent [pour détection] défini en forme d'une couche et/ou d'une imprégnation est distribué d'une manière non-homogène et que l'agent existe d'une quantité prédéterminé dans un part prédéterminé, des fibres formant le matériau de fibre, et que dans un autre part prédéterminé des fibres formant le matériau de fibre l'agent n'existe pas du tout ou d'une quantité différente du premier part prédéterminé des fibres formant le matériau de fibre.

3. Détecteur selon la Revendication 1, **caractérisé en ce que** un ou plusieurs des agents [pour détection] sont dans une distribution homogène définie sont contenus sous une ou plusieurs formes d'imprégnation et/ou de couchage de fibres.

4. Détecteur selon une des Revendications 1 à 3, **caractérisé en ce que** la caisse consiste en une des apparences : filet, feuille, textile, tissu, toison, papier, non-tissé, tuyau perforé, matériau composite, tricotage, grille en fils de fer, tôle perforée.

5. Détecteur selon une des Revendications 1 à 4, **caractérisé en ce qu'**il contient des substances accessoires activables par des champs, activable par au moins une des actions de champ : champ de gravitation, champ magnétique, et champ électrique.

6. Détecteur selon une des Revendications 1 à 5, **caractérisé en ce qu'**il contient un dispositif qui a au moins une des fonctions de la groupe suivante : tenir ensemble le contenu du détecteur, protection du contenu du détecteur contre décomposition, formage, conduction d'écoulement, force ascensionnelle, force descensionnelle, isolation électrique, emboîtement des composants du détecteur.

7. Procédé dans un système ouvert pour détecter le genre et/ou le contenu des substances prédéterminées et/ou pour déterminer des paramètres de direction à un lieu d'examen dans des eaux où pendant la durée de l'examen le milieu passe pendant plus d'une heure passivement par ou autour d'un élément volumétrique d'un détecteur selon une des Revendications 1 à 6, **caractérisé en ce que** par échantillonnage passive le détecteur est placé dans le milieu examiné pour y rester pendant la durée d'examen après laquelle le détecteur est retiré pour alors déterminer la quantité du au moins un agent et/ou du produit de réaction formé par l'action de la substance prédéterminé et/ou l'écoulement du courant du liquide sur le du moins un agent, mesurant ainsi le genre et, le cas échéant, la quantité de la substance prédéterminée et/ou de l'écoulement du courant du liquide.

8. Procédé selon la Revendication 7, **caractérisé en ce qu'**un détecteur est employé qui se compose de plusieurs composants filiformes ou sous forme de ruban empreints d'un mouvement relatif au milieu fluide à mesurer.

9. Procédé selon une des Revendications 7 et 8, **caractérisé en ce que** dans le détecteur, ou bien du moins dans sa sphère d'influence est, en addition aux paramètres de courant ou au lieu des paramètres de courant existants, généré dans le compartiment d'examen un courant de liquide déviant des paramètres de courant existants, ou un mouvement du milieu examiné relativement au détecteur.

10. Usage du détecteur selon une des Revendications 1 à 6 dans un système ouvert pour déterminer le contenu des substances prédéterminées et/ou pour déterminer des paramètres de direction er/ou de vitesse dans des eaux, le milieu étant un aquifère naturel ou artificiel, des eaux souterraines, un canal, un lac de barrage, des eaux courantes, un lac intérieur, un égout, une installation de traitement des eaux, un port, ou une mer.
